(19) Europäisches Patentamt
European Patent Office
Office européen des brevets

(11) **EP 3 630 197 B1**

(12) **EUROPEAN PATENT SPECIFICATION**

(45) Date of publication and mention
of the grant of the patent:
**16.08.2023 Bulletin 2023/33**

(21) Application number: **18732253.2**

(22) Date of filing: **22.05.2018**

(51) International Patent Classification (IPC):
**C07K 14/805** (2006.01)   **C12N 5/0789** (2010.01)
**A61K 48/00** (2006.01)

(52) Cooperative Patent Classification (CPC):
**C12N 5/0647; C07K 14/805;** A61K 48/005;
C12N 2501/999; C12N 2510/00; C12N 2740/16043

(86) International application number:
**PCT/EP2018/063395**

(87) International publication number:
**WO 2018/215473 (29.11.2018 Gazette 2018/48)**

(54) **METHOD OF SELECTING GENETICALLY MODIFIED HEMATOPOIETIC STEM CELLS**

VERFAHREN ZUR AUSWAHL VON GENETISCH MODIFIZIERTEN HAEMATOPOIETISCHEN
STAMMZELLEN

PROCÉDÉ DE SÉLECTION DE CELLULES SOUCHES HEMATOPOÉTIQUES MODIFIÉES
GÉNÉTIQUEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.05.2017 EP 17305598**

(43) Date of publication of application:
**08.04.2020 Bulletin 2020/15**

(73) Proprietors:
• **Commissariat à l'Énergie Atomique
et aux Énergies Alternatives
75015 Paris (FR)**
• **INSERM (Institut National de la Santé
et de la Recherche Médicale)
75013 Paris (FR)**
• **Université Paris-Saclay
91190 Saint-Aubin (FR)**

(72) Inventors:
• **PAYEN, Emmanuel
92410 Ville d'Avray (FR)**
• **BHUKHAI, Kanit
75014 Paris (FR)**
• **LEBOULCH, Philippe
Bangkok 10600 (TH)**
• **DOWN, Julian
Cambridge, MA 02139 (US)**

(74) Representative: **Plasseraud IP
66, rue de la Chaussée d'Antin
75440 Paris Cedex 09 (FR)**

(56) References cited:
• **K DORMIANI ET AL: "Long-term and efficient
expression of human [beta]-globin gene in a
hematopoietic cell line using a new site-specific
integrating non-viral system", GENE THERAPY,
vol. 22, no. 8, 1 April 2015 (2015-04-01), pages
663-674, XP055393985, GB ISSN: 0969-7128, DOI:
10.1038/gt.2015.30**
• **JEAN-ANTOINE RIBEIL ET AL: "Gene Therapy in
a Patient with Sickle Cell Disease", NEW
ENGLAND JOURNAL OF MEDICINE, THE - NEJM
-, vol. 376, no. 9, 2 March 2017 (2017-03-02), pages
848-855, XP055393852, ISSN: 0028-4793, DOI:
10.1056/NEJMoa1609677**
• **OLIVIER NEGRE ET AL: "Gene Therapy of the
[beta]-Hemoglobinopathies by Lentiviral
Transfer of the [beta] A(T87Q) - Globin Gene",
HUMAN GENE THERAPY, vol. 27, no. 2, 22
January 2016 (2016-01-22), pages 148-165,
XP055393979, US ISSN: 1043-0342, DOI:
10.1089/hum.2016.007**

EP 3 630 197 B1

- T LICHT ET AL: "Drug selection of MDR1-transduced hematopoietic cells ex vivo increases transgene expression and chemoresistance in reconstituted bone marrow in mice", GENE THERAPY, vol. 7, no. 4, 1 January 2000 (2000-01-01), pages 348-358, XP055393769, DOI: 10.1038/sj.gt.3301087
- THEILE D ET AL: "ATP-binding cassette transporters as pitfalls in selection of transgenic cells", ANALYTICAL BIOCHEMISTRY, ELSEVIER, AMSTERDAM, NL, vol. 399, no. 2, 15 April 2010 (2010-04-15), pages 246-250, XP026924496, ISSN: 0003-2697, DOI: 10.1016/J.AB.2009.12.014 [retrieved on 2010-02-24]
- Murtaza S Nagree ET AL: "Towards in vivo amplification: Overcoming hurdles in the use of hematopoietic stem cells in transplantation and gene therapy INTRODUCTION", World J Stem Cells December, 26 December 2015 (2015-12-26), pages 1233-1250, XP055393767, DOI: 10.4252/wjsc.v7.i11.1233 Retrieved from the Internet: URL:https://www.ncbi.nlm.nih.gov/pmc/artic les/PMC4691692/pdf/WJSC-7-1233.pdf [retrieved on 2017-07-25]
- JI Vollweiler ET AL: "Mini review Hematopoietic stem cell gene therapy: progress toward therapeutic targets", , 1 January 2003 (2003-01-01), XP055393845, Retrieved from the Internet: URL:http://www.nature.com/bmt/journal/v32/ n1/pdf/1704081a.pdf [retrieved on 2017-07-26]
- Olivier Negre ET AL: "Preclinical Evaluation of Efficacy and Safety of an Improved Lentiviral Vector for the Treatment of -Thalassemia and Sickle Cell Disease", Current Gene Therapy, vol. 15, 1 January 2015 (2015-01-01), pages 64-81, XP055393858, DOI: 10.2174/1566523214666141127095336

Remarks:

The complete document including Reference Table(s) and the Sequence Listing(s) can be downloaded from the EPO website

**Description**

The present invention pertains to the field of hematopoietic stem cell gene therapy. The present invention relates to a method of selecting genetically modified hematopoietic stem cells, and its use for hematopoietic stem cell gene therapy.

Gene therapy *via* the *ex vivo* genetic modification of hematopoietic cells including hematopoietic stem cells (HSCs), with a transfer vector that drives expression of a therapeutic polypeptide, followed by reinjection of the resulting genetically modified cells into a patient, offers great potential for the treatment of numerous hematologic and non-hematologic genetic and non-genetic disorders.

Although gene transfer to hematopoietic stem cells (HSCs) has shown therapeutic efficacy for several individuals with inherited disorders in recent trials, gene transfer incompleteness of the HSC population remains a hurdle to yield a cure for all patients.

Gene transfer is a limiting step in HSC gene therapy, which necessitates the use of numerous vector particles to modify only few cells. While increasing the number of vectors integrating into the genome is an attractive approach for increasing the proportion of vector-bearing HSCs and the probability of the therapeutic gene being expressed, safety concerns remain, due to the risk of insertional mutagenesis.

In view of improving hematopoietic stem cell gene therapy, numerous methods for selecting genetically modified hematopoietic cells including stem cells have been proposed but failed to show practical utility for human clinical applications.

A first type of *in vitro* selection methods are based on (i) co-expression of a membrane marker with the gene of interest or expression of a fluorescent protein, using the gene transfer vector, and (ii) isolation of genetically modified hematopoietic cells including stem cells by magnetic-activated-separation (MACS) or fluorescent activated cell sorting (FACS), before transplantation. These approaches are limited by the impracticability of isolating cells expressing fluorescent proteins for human use and the major loss of cells during the physical manipulation of the primary cells that are fragile, especially HSCs, and cannot withstand lengthy and/or traumatic physical manipulations.

*In vivo* selection methods are based on (i) co-expression of a selectable marker that confers resistance to a toxic drug, with the gene of interest, using the gene transfer vector, and (ii) administering the respective cytotoxic drug, after transplantation. The selectable marker is Multidrug resistance 1 (MDR1), Dihydrofolate reductase (DHFR) or Methylguanine-DNA-Methyltransferase (MGMT) gene. These approaches, however, are limited by undesirable hematological toxicity and myelosuppressive complications from the treatment. In addition, MGMT-based selection is not safe because the use of a genotoxic drug (carmustine (BCNU), nimustine (ACNU) or temozolomide (TMZ)) for the selection may result in mutations that could favor the uncontrolled amplification of hematopoietic clones bearing provectors near/in oncogenes and thus increase the risk of insertional mutagenesis as suggested in a clinical trial (Adair et al., Sci. Transl. Med., 2012, 4, 133ra57). Moreover, DHFR-based selection, using for example methotrexate (MTX) as selection agent does not allow the selection of genetically modified HSCs in nonhumate primate models (Persons et al., Blood, 2004, 103, 796) and MDR1 expression has led to leukemogenesis in mice after selection of clones with multiple insertion sites, throwing suspicion on the usability of MDR1 as a selective agent.

Another type of *in vitro* selection methods is based on (i) co-expression of a selectable marker that confers resistance to antibiotics (neomycin, hygromycin, puromycin) with the gene of interest, using the gene transfer vector, and (ii) adding the respective antibiotics to the cell culture, usually 10 days at minimum. These methods are limited by the loss of viability and engraftment potential of the cells after such lengthy culture *in vitro.* It is admitted that the culture of the cells *in vitro* for so many days is poorly compatible with sufficient maintenance of cell viability and state of differentiation of many primary cell types to be used in gene therapy protocols. Hence, hematopoietic stem cell populations submitted to this approach lose their engraftment potential in transplanted recipients.

Dormiani et al. (Gene Therapy, 2015, 22, 663-674) discloses the selection of an immortalized human erythroid (K562) cell line expressing beta-globin using a site-specific non-viral integrating non-viral system and puromycin selection. Ribeil et al. (New England Journal of Medicine, 2017, 376, 848-855) and Negre et al. (Human Gene Therapy, 2016, 27, 148-165) disclose the treatment of beta-hemoglobinopathies by ex-vivo transduction of patient's CD34+ hematopoietic cells (HSCs) with a beta-globin-expressing lentiviral vector without selection of the transduced HSCs. Licht et al. (Gene therapy, 2000, 7, 348-358) discloses in vivo selection of genetically modified HSCs by transduction with MDR1 expressing vector in the presence of a cytotoxic agent (colchicine, daunomycin). Theile et al. (Analytical Biochemistry, 2010, 239, 246-250) shows that overexpression of MDR1 in an immortalized cell line stably transfected with MDR1 increases puromycin resistance.

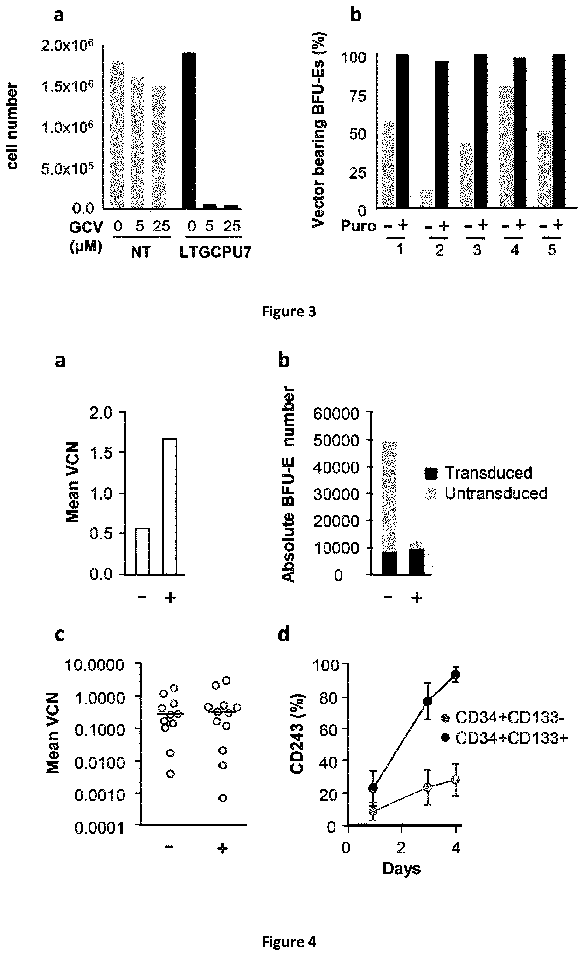

WO 2012/094193 discloses a method of enhancing the reconstitution by transduced hematopoietic cells in a transplant recipient comprising selecting transduced hematopoietic cells *in vitro* using puromycin at a concentration of 1-25 μg/ml for 1 to 4 days. However, the inventors have shown that this method is not suitable for hematopoietic stem cell gene therapy because human hematopoietic stem cells cannot be selected with puromycin **(Figure 4c).**

To improve the efficacy of current gene transfer methods, there is a need for new gene transfer methods that increase the proportion of genetically modified hematopoietic stem cells. In addition, to improve the efficacy as well as the safety of current gene transfer methods, it would be most desirable to have a method that increases the proportion of genetically modified hematopoietic stem cells without also increasing the number of copies of the vector per cell.

The inventors have shown that the lack of selection of genetically modified human hematopoietic stem cells (HSCs) with puromycin was correlated with high level MDR1 expression on HSCs (**Figure 4d**) which could cause the lack of selection. This hypothesis was confirmed by showing that complete selection and preservation of genetically modified human hematopoietic stem cells (HSCs) were achieved after brief exposure to puromycin in the presence of a MDR1 inhibitor. HSCs were efficiently selected in the presence of MDR1 inhibitor (**Figure 6**) and transplanted with a minimum loss of *in vivo* HSC activity (**Figure 7**), suggesting the procedure's suitability for human clinical applications.

The present invention relates to a method of selecting genetically modified hematopoietic stem cells, comprising the steps of:

a) co-delivering at least : (i) a polynucleotide of interest and/or a genome-editing enzyme and (ii) a positive selection marker which is puromycin N-acetyl transferase or a polynucleotide encoding said marker in expressible form, into a population of CD34+ hematopoietic cells, and
b) contacting the population of hematopoietic cells obtained in a) with an agent for selecting the marker in a) and with a multidrug resistance 1 (MDR1) inhibitor, wherein the agent is puromycin, thereby selecting genetically modified hematopoietic stem cells.

The combined use of the selection agent which is puromycin and the MDR1 inhibitor in step b) of the method of the invention allows to eliminate unmodified hematopoietic cells (in which the co-delivery in step a) has not occurred) with the selection agent and to select genetically modified hematopoietic stem cells (being genetically modified in their genome by the polynucleotide of interest) with the selection agent plus MDR1 inhibitor. Therefore, the population of genetically modified hematopoietic cells resulting from step b) of the method of the invention comprises a higher proportion of genetically modified hematopoietic stem cells compared with the corresponding population of genetically modified hematopoietic cells obtained by performing step b) only with the selection agent, as in the prior art method.

## Definitions

As used herein, the term "hematopoietic cells" refers to cells produced by the differentiation of hematopoietic stem cells (HSCs or HSC). Hematopoietic cells include HSCs, multipotent and lineage-committed progenitors, precursor cells and mature cells. Mature hematopoietic cells include with no limitations, lymphocytes (B, T), NK cells, monocytes, macrophages, granulocytes, erythrocytes, platelets, plasmacytoid and myeloid dendritic cells, and microglial cells.

As used herein, the term "hematopoietic stem cells (HSCs)" refers to self-renewing cells capable of reconstituting short or long-term hematopoiesis following transplantation.

As used herein, the term "genetic modification" refers to the insertion, deletion, and/or substitution of one or more nucleotides into a genomic sequence.

As used herein, the term "HSC gene therapy" refers to the transfer of a polynucleotide sequence of interest into HSCs, in order to obtain genetically modified HSCs useful for the treatment and/or the prevention of inherited or acquired diseases.

As used herein, the term "MDR1" refers to ABCB1, the first member of ATP-binding cassette (ABC) transporter subfamily B genes (ABCB), also known as MDR/TAP subfamily. Human MDR1 gene encodes P-glycoprotein (P-gp or P170) which is an ATP-dependent drug efflux pump.

The polynucleotide of interest according to the method of the invention is a polynucleotide that modify the genome of hematopoietic stem cells to generate genetically modified hematopoietic stem cells. The polynucleotide may be DNA, RNA or a synthetic or semi-synthetic nucleic acid.

The genome-editing enzyme according to the method of the invention is an enzyme or enzyme complex that induces a genetic modification at a target genomic locus. The genome-editing enzyme is advantageously an engineered nuclease which generates a double-strand break (DSB) in the target genomic locus, such as with no limitations, a meganuclease, zinc finger nuclease (ZFN), transcription activator-like effector-based nuclease (TALENs) and clustered regularly interspaced palindromic repeats (CRISPR)-Cas system. The genome-editing enzyme, in particular an engineered nuclease, is usually but not necessarily used in combination with a homologous recombination (HR) matrix or template (also named DNA donor template) which modifies the target genomic locus by double-strand break (DSB)-induced homologous recombination. In particular, the HR template may introduce a transgene of interest into the target genomic locus or repair a mutation in the target genomic locus, preferably in a gene of interest.

The polynucleotide of interest may comprise a sequence encoding a protein and/or an RNA of interest (*i.e.,* a transgene of interest) in expressible form. In some embodiments, the polynucleotide of interest comprises a sequence encoding a protein of interest in expressible form.

The polynucleotide of interest may comprise a sequence which modifies a gene of interest in the genome of HSCs, in particular a sequence which repairs a mutation in said gene of interest. In some embodiments, the polynucleotide of interest comprises a sequence which repairs a mutation in said gene of interest.

The polynucleotide of interest may be a homologous recombination template that is used in combination with a genome-editing enzyme, in particular an engineered nuclease, as defined above. The homologous recombination template comprises advantageously a nucleotide sequence encoding a protein of interest or a nucleotide sequence which modifies a gene of interest, as defined above. The method of the invention may be performed using hematopoietic cells including stem cells obtained from a variety of sources, using conventional methods known and available in the art. For example, hematopoietic cells may be recovered from bone marrow, mobilized peripheral blood mononuclear cells (PBMCs), umbilical cord blood, embryonic stem (ES) cells or induced pluripotent stem (iPS) cells. Peripheral blood stem cell mobilization is usually performed using granulocyte colony-stimulating factor (G-CSF) and/or plerixafor. Hematopoietic cells may be purified or selected, for example based upon cell surface marker(s) that are differentially expressed on primitive cells (hematopoietic stem and progenitor cells (HSPCs)) and more differentiated cells. The most important marker of primitive human hematopoietic cells is CD34. Other most common markers include Lin which is absent on HSPCs, CD38 and CD45RA which are absent or weakly expressed on primitive cells, CD90 (Thy-1) which is expressed at higher levels on primitive cells than on differentiated cells, CD133 which is expressed on HSCs, or combination thereof. HSPCs are purified using at least one antibody directed to a marker, by standard antibody-based cell isolation methods such as magnetic-activated-separation (MACS) or fluorescence activated cell sorting (FACS).

The population of CD34+hematopoietic cells, preferably human CD34+hematopoietic cells, is advantageously obtained from bone marrow, mobilized peripheral blood mononuclear cells or umbilical cord blood.

The method of the invention can be carried out *in vitro* using culture media and conditions suitable for the maintenance, growth or proliferation of hematopoietic cells including stem cells. Suitable culture media and conditions are well known in the art.

The method of the invention uses standard nucleic acid and/or protein delivery agents or systems. The method of the invention may be performed using chemical or physical delivery methods or a combination thereof. Chemical methods include the use of particles, lipids, polymers, vectors and combination thereof. For example, the method of the invention may use microparticles, nanoparticles, lipid particles, nanocomplexes, liposomes, cationic polymers, and vectors. Physical methods include with no limitations electroporation, microinjection and magnetofection.

In some embodiments of the method of the invention, step a) comprises the co-delivery of at least a genome-editing enzyme and a positive selection marker, preferably with a polynucleotide of interest as defined above.

In some other embodiments of the method of the invention, step a) comprises the co-delivery of at least a polynucleotide of interest and a positive selection marker. In some other embodiments of the method of the invention, step a) comprises the co-delivery of at least a polynucleotide of interest and a polynucleotide encoding the positive selection marker in expressible form.

The polynucleotide of interest and/or the polynucleotide encoding the selection marker are advantageously introduced into a vector. The two polynucleotides may be introduced in the same vector or in separate vectors. Recombinant transfer vector(s), gene transfer vector(s) or transfer vector(s) which can be used in the present invention includes, in a non-limiting manner, linear or circular DNA or RNA molecules consisting of chromosomal, non-chromosomal, synthetic or semi-synthetic nucleic acids, such as in particular viral vectors, plasmid or RNA vectors. Numerous vectors into which a nucleic acid molecule of interest can be inserted in order to introduce it into and maintain it in a eukaryotic host cell including hematopoietic cell are known *per se*; the choice of an appropriate vector depends on the use envisioned for this vector (for example, replication of the sequence of interest, expression of this sequence, maintaining of this sequence in extrachromosomal form, or else integration into the chromosomal material of the host), and also on the nature of the host cell.

Naked nucleic acid vector such as plasmid is usually combined with a substance which allows it to cross the host cell membrane, such as a transporter, for instance a nanotransporter or a preparation of liposomes, or of cationic polymers. Alternatively, the naked nucleic acid may be introduced into said host cell using physical methods such as electroporation or microinjection. In addition, these methods can advantageously be combined, for example using electroporation combined with liposomes.

Viral vectors are by nature capable of penetrating into cells and delivering polynucleotide(s) of interest into cells, according to a process named as viral transduction. Therefore, the polynucleotide sequence(s) according to the invention are usually introduced into cells by contacting the recombinant viral vector with said cells. Sometimes, viral transduction may be combined with physical methods such as for example magnetofection.

Viral vectors include those derived from retroviridae, adenoviridae, parvoviridae, poxviridae (retrovirus, adenovirus, adeno-associated virus (AAV), herpes virus, poxvirus), and other virus vectors. Retrovirus includes in particular gammaretrovirus, spumavirus, and lentivirus. Lentivirus includes in particular human immunodeficiency virus, including HIV type 1 (HIV1) and HIV type 2 (HIV2), feline immunodeficiency virus (FIV), bovine immunodeficiency virus (BIV), equine immunodeficiency virus (EIV), simian immunodeficiency virus (SIV), visna-maedi and caprine arthritis-encephalitis virus (CAEV), Jembrana disease virus, and Puma lentivirus.

The recombinant vectors are constructed using standard recombinant DNA technology techniques and produced using conventional methods that are known in the art.

In some embodiments, the method uses at least one vector suitable for stable gene transfer and long-term gene expression into mammalian cells, such as by replication of the sequence of interest, expression of this sequence, maintaining of this sequence in extrachromosomal form, or else integration into the chromosomal material of the host. Such vector is usually used to perform the delivery of the polynucleotide of interest in the absence of genome-editing enzyme. The polynucleotide of interest comprises advantageously a transgene of interest in expressible form. The delivery of the polynucleotide sequence encoding the selection marker may also be performed using the same type of vector, wherein the two polynucleotides are in the same vector or in two separate vectors. The at least one vector(s) for stable gene transfer and long-term gene expression into mammalian cells is advantageously an integrating vector, in particular an integrating viral vector such as a retrovirus or AAV vector. Preferably, the vector is a lentiviral vector. The lentivirus vector is advantageously a self-inactivating vector (SIN vector). The lentiviral vector comprises advantageously a central polypurine tract/DNA FLAP sequence (cPPT-FLAP), and/or insulator sequence(s) such as chicken beta-globin insulator sequence(s) to improve expression of the gene(s) of interest. The lentiviral vector is advantageously pseudotyped with another envelope protein, preferably another viral envelope protein, preferably the vesicular stomatis virus (VSV) glycoprotein. Preferred lentiviral vector is a human immunodeficiency virus (HIV) vector.

The delivery of the polynucleotide of interest, when performed in the presence of a genome editing enzyme, and/or the delivery of the positive selection marker may use transient expression vector(s), in particular plasmid vector(s).

In some embodiments, the polynucleotide of interest, preferably comprising a transgene of interest in expressible form, is inserted in an integrating vector as defined above, preferably a lentiviral vector. The polynucleotide encoding the selection marker may be inserted in the same vector or in another vector such as a transient expression vector, in particular a plasmid vector, as defined above.

The recombinant transfer vector used for the delivery of the transgene of interest and/or the polynucleotide encoding the selection marker is an expression vector. The expression vector comprises appropriate means for expression of said protein of interest and/or said selection marker in hematopoietic cells including stem cells. Usually, each coding sequence (protein of interest and selection marker) is inserted in a separate expression cassette. Each expression cassette comprises the coding sequence (open reading frame or ORF) functionally linked to the regulatory sequences which allow the expression of the corresponding protein (protein of interest or selection marker) in the host cell, such as in particular promoter, promoter/enhancer, initiation codon (ATG), codon stop, transcription termination signal. Alternatively, the protein of interest and the selection marker may be expressed from a unique expression cassette using an Internal Ribosome Entry Site (IRES) inserted between the two coding sequences or a viral 2A peptide. The coding sequences of the selection marker and protein of interest may also be fused in frame to produce a fusion protein. In addition, the codon sequence encoding the protein of interest and/or the selection marker is advantageously optimized for expression in host cells, in particular in human cells.

The promoter may be a tissue-specific, ubiquitous, constitutive or inducible promoter that is functional in hematopoietic cells including stem cells. Examples of constitutive promoters which can be used in the present invention include with no limitations: phosphoglycerate kinase promoter (PGK), elongation factor-1 alpha (EF-1 alpha) promoter including the short form of said promoter (EFS), viral promoters such as cytomegalovirus (CMV) immediate early enhancer and promoter, and retroviral 5' and 3' LTR promoters including hybrid LTR promoters. An example of tissue-specific promoter which can be used in the present invention is the beta-globin promoter which is expressed exclusively in erythroid cells. Examples of inducible promoters which can be used in the present invention include Tetracycline-regulated promoters. The promoters are advantageously human promoters, *i.e.,* promoters from human cells or human viruses. Such promoters are well-known in the art and their sequences are available in sequence data base.

The vector comprising the polynucleotide of interest, and eventually also the polynucleotide encoding the selection marker, may further comprise the polynucleotide sequence of a conditional suicide gene such as Herpes simplex virus thymidine kinase (HSV-TK) gene or a functional variant thereof. For example, the conditional suicide gene is a deleted version of HSV-TK starting at the second ATG. The conditional suicide gene is advantageously fused in frame with the selection marker gene. In some advantageous embodiments, the protein of interest is a therapeutic protein, which means a protein that provides a therapeutic effect in the treatment and/or the prevention of inherited or acquired disease. The therapeutic protein may be a protein which corrects a genetic deficiency in hematopoietic stem cells and/or their progeny for the treatment of hematologic and non-hematologic genetic disorders or a protein which provide hematopoietic stem cells and/or their progeny with new properties or enhanced properties for the treatment of genetic or non-genetic diseases.

The polynucleotide encoding the protein of interest is advantageously under the control of a promoter which is specific for the target hematopoietic cell(s), such as the beta-globin promoter which is specific for erythroid cells.

For corrective stem cell gene therapy, the polynucleotide sequence according to the invention comprises a functional copy of a gene (*i.e.* encoding a functional protein) to correct a mutated gene causing the deficiency associated with the genetic disease. Therapeutic targets of corrective stem cell gene therapy are known in the art and include with no limitations: Primary immunodeficiencies such as Adenosine Deaminase deficiency (ADA gene), X-linked SCID syndrome (common gamma chain (gamma-c) gene), Chronic granulomatous diseases (CGD; p47-PHOX, p91-PHOX/p67-PHOX genes), X-linked agammaglobulinemia (BTK gene) and Wiskott-aldrich syndrome (WAS gene); Hemoglobinopathies such as Sickle-cell disease (beta-globin or HBB gene), Beta thalassemia (beta-globin or HBB gene) and Alpha thalassemia (alpha-globin (HBA1 or HBA2) gene); other single-gene disorders such as Lysosomal storage disorders like for example Hurler's disease (IDUA gene coding for lysosomal alpha-L-idurodinase), Gaucher's disease (GBA gene coding for beta-glucocerebrosidase), Farber's disease (Lysosomal acid ceramidase (ASAH gene) and Fabry's disease (alpha-galactosidase (GLA gene); Neurological storage diseases such as leukodystrophies, for example Adrenoleukodystrophy (ABCD1 gene coding for the adrenoleukodystrophy protein (ALDP) and Metachromatic leukodystrophy (ARSA gene coding for arylsulfatase A enzyme); Hemophilia A (F8 gene coding for coagulation factor VIII) and Hemophilia B (F9 gene coding for coagulation factor IX); Alpha-antitrypsin deficiency (SERPINA1 gene) and Stem cell defects such as Fanconi anemia (FA pathway genes, in particular FANCA, FANCC and FANCG).

In some embodiments, the therapeutic protein is beta-globin, preferably human beta-globin.

Treatment of non-genetic diseases includes for example, cancer, cardiovascular diseases, auto-immune diseases and infectious diseases. Therapeutic proteins for cancer therapy include tumor-associated antigens, cytokines and chemokines, chemotherapy resistance genes (MDR-1, DHFR, MGMT), tumor suppressor genes. A therapeutic protein for cardiovascular diseases is for example VEGF. Infectious diseases include in particular HIV infection. Treatment of infectious diseases like HIV includes providing hematopoietic progeny cells resistant to infection in particular lymphocytes (HIV) resistant to infection.

As known in the art, the positive selection marker puromycin N-acetyl transferase (PAC) is a protein which confers an advantage to cells expressing said protein as this enzyme confers resistance to the antibiotic puromycin, a toxic drug that is used as selection agent. The selection agent is used to kill unmodified cells (*i.e.* cells in which (i) the polynucleotide sequence of interest and/or genome-editing enzyme, and (ii) the positive selection marker or polynucleotide sequence encoding said marker have not been delivered).

The positive selection marker puromycin N-acetyl transferase (PAC) is an enzyme conferring resistance to an antibiotic (puromycin) that is a MDR1 substrate. According to the invention, a MDR1 substrate refers to an antibiotic for which MDR1 expression confers resistance to cells. Puromycin is usually used at a concentration of 1-25 $\mu$g/ml, preferably about 5 $\mu$g/ml.

The selection marker is advantageously under the control of a constitutive ubiquitous promoter such as for example phosphoglycerate kinase promoter (PGK) or elongation factor-1 alpha (EF-1 alpha) promoter including the short form of said elongation factor promoter (EFS). In some preferred embodiments, the selection marker is under the control of EFS promoter, preferably human EFS promoter.

The multidrug resistance 1 (MDR1) protein inhibitor may be an inhibitor of MDR1 gene expression or an inhibitor of MDR1 protein function. The P-gp inhibitor may act as a competitive blocker via occupying the drug binding sites, as a non-competitive antagonist or as an agent inhibiting the ATPase function.

Examples of MDR1 inhibitors include antisens nucleic acid molecules, interfering nucleic acid molecules such as siRNA, miRNA or shRNAs and ribozymes targeting the MDR1 gene, anti-MDR1 antibodies and other molecules or compounds which block or inhibit the activity of MDR1 protein, i.e., the drug efflux/transport function of MDR1 protein.

MDR1 inhibitors are known in the art and include first, second or third generation inhibitors. Non-limiting examples of such inhibitors include verapamil, dexverapamil, emopamil, gallopamil, Roll-2933, diltiazem, bepridil, nicardipine, nifedipine, felodipine, isradipine, trifluorperaine, clopenthixol, trifluoupromazine, flupenthixol, chlorpromazine, prochlorperazine, indol alkaloids, reserpine, mifepristone, cyclosporin-A, valspodar (PSC 883), quinine, tariquidar, elacridar, zosuquidar, timcodar, curcumin, phenyl cinnamate, coumarin, apiole, bergamottin beta-amyrin cinnamate, caffeine, caffein 8-decylthio, caffein 8-benzyl, diethylpyrocarbonate, morin, narirutin, piperin, quercetin, slavironin, silybin, theobromin, vanillin, vanillin-N-nonlymine, zosuquidar, minocycline, reversan, amiodarone, azithromycin, captopril, clarithromycin, piperine, quercetin, quinidine, ritonavir and derivatives thereof.

In a particular embodiment of the method of the invention, said MDR1 inhibitor is selected from the group consisting of: cyclosporin-A, verapamil, reserpine, mifepristone and derivatives thereof. In a preferred embodiment of the method of the invention, the positive selection marker is puromycin resistance and said MDR1 inhibitor is selected from the group consisting of: cyclosporin A, verapamil, reserpine and mifepristone. In a more preferred embodiment, said MDR1 inhibitor is cyclosporin-A.

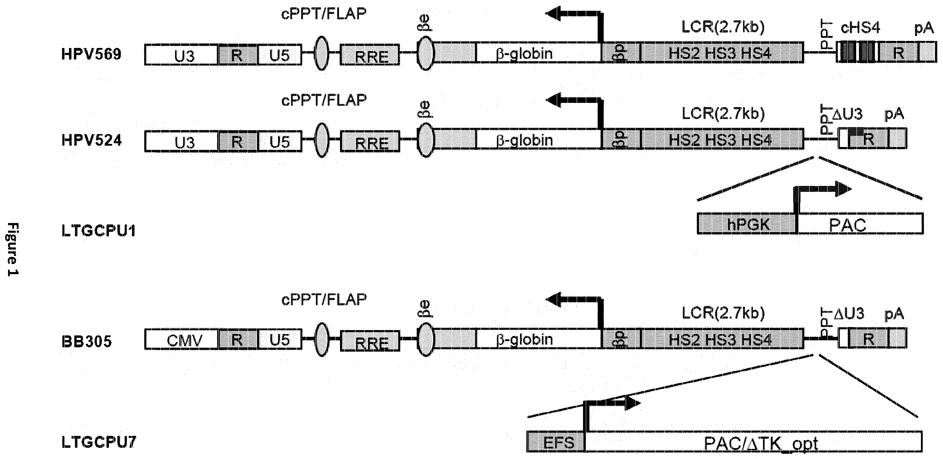

. In a more preferred embodiment, step a) of the method of the invention is performed using a lentivirus vector comprising a polynucleotide of interest encoding a therapeutic protein and a polynucleotide encoding puromycin N-acetyl transferase (PAC) in expressible form. Non-limiting examples of such lentiviral vectors based on puromycin resistance are the lentiviral vectors named LTGCPU1 and LTGCPU7 shown in figure 1.

According to the invention, said method is carried out in a period of time inferior to one week, preferably in four days or less. This period is compatible with the clinical protocols of HSC gene therapy with retroviral or lentiviral vector.

The first step is generally carried out for 24-72 hours, preferably 48 hours, and the second step of contacting the cells with said selection marker and a MDR1 inhibitor is generally carried out for 24-48 hours, preferably 24 hours. The cells may be optionally cultured in appropriate culture medium, before step a) (prestimulation step), and/or after step b). The prestimulation step is generally carried out for 16-24 hours. The population of genetically modified hematopoietic stem cells obtained at the end of step b) is frozen for future use or transplanted into patients in need of HSC gene therapy.

Therefore, the method of the invention is useful in HSC gene therapy for treating genetic and non-genetic diseases as defined above.

Other diseases include for example IPEX Syndrome, Hemophagocytic Lymphohistiocytosis (HLH), X-linked Lymphoproliferative Disease (XLP), blood cancer (leukemia), lymphoma, bone marrow cancer, myeloma, protoporphyria, pathologies associated with Leukocyte Adhesion Deficiency (LAD), HIV disease, Multiple sclerosis, Systemic scleroderma, Crohn's disease and Lupus erythematosus.

Said disease is advantageously a genetic hematologic or non-hematologic disorder, in particular a hemoglobinopathy, more particularly Sickle-cell disease or beta thalassemia.

In addition to the preceding description, the invention also comprises other arrangements that will emerge from the description which follows, which refers to exemplary embodiments of the method which is the subject of the present invention and also the appended drawings.

The practice of the present invention will employ, unless otherwise indicated, conventional techniques, which are within the skill of the art. Such techniques are explained fully in the literature.

For a better understanding of the invention and to show how the same may be carried into effect, there will now be described by way of example a specific mode contemplated by the Inventors with reference to the accompanying drawings in which:

- **Figure 1: Schematic overview of the lentiviral vector constructs used in this study and the parental vectors.** All the LVs used encoded the beta$^{AT87Q}$-globin chain under the control of the beta-globin promoter and hypersensitive sites (HS) of the beta-globin locus control region (LCR) and were derived from the HPV569 and BB305 lentiviral beta-globin vector that have been tested in approved human trials for the gene therapy of beta-hemoglobinopathies (Negre, O. et al., Human gene therapy, 2016, 27, 148-165). The Tat-dependent HPV569, HPV524 and LTGCPU1 vectors contain a natural 5' long terminal repeat from HIV, whereas BB305 and LTGCPU7 contain a cytomegalovirus (CMV) promoter and enhancer instead of the HIV U3 region. In LTGCPU1 and LTGCPU7, the human phosphoglycerate kinase 1 (hPGK) promoter or the short-form human elongation factor (EF)-1alpha (EFS) promoter controls expression of the puromycin N-acetyl-transferase (PAC) gene and a deleted version of the human herpes 1 thymidine kinase gene (deltaTK) starting at the second ATG. The products of the PAC and TK open reading frames are fused through a two-amino acid glycine-serine linker. In LTGCPU7, the codon sequence encoding the PAC/deltaTK fusion protein has been modified (_opt) to optimize expression in human cells.

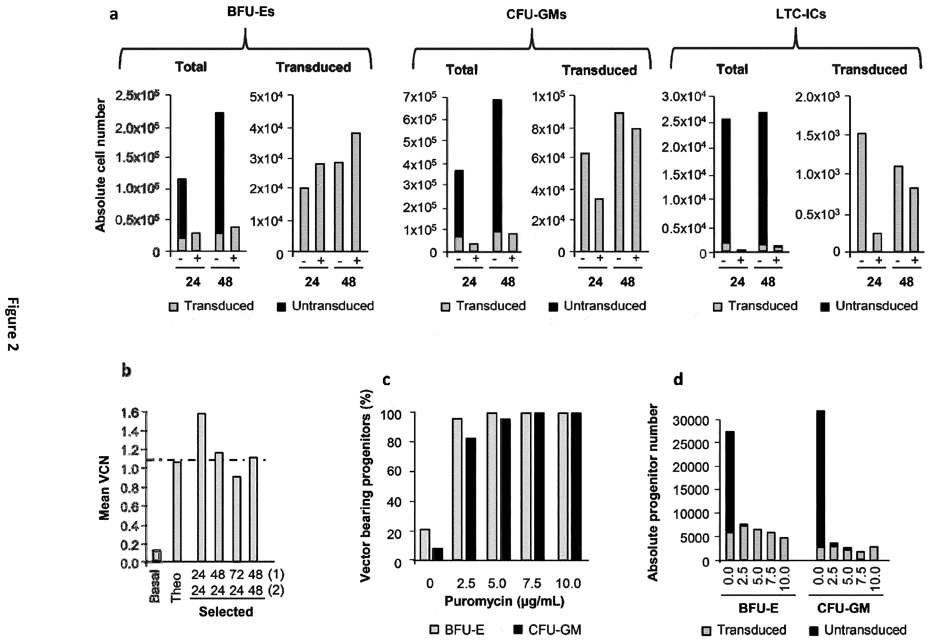

- **Figure 2: Efficient selection and recovery of transduced progenitor cells**

  **a.** Adult bone marrow cells were transduced with LTGCPU1. 24 or 48 hours later, cells were left untreated (-) or treated (+) with puromycin (10 $\mu$g/mL) for a further 24 hours. The cells were then immediately plated on semi-solid medium or grown in liquid culture for the evaluation of erythroid (BFU-Es) and myeloid (CFU-GM) progenitor cell numbers and to determine the number of long-term culture-initiating cells (LTC-IC). At the end of the culture period, colony-forming cells were counted and picked for qPCR analyses of the presence of the vector. The absolute numbers of transduced (grey) and non-transduced (black) progenitor cells are indicated.
  **b.** Mobilized bone marrow cells were transduced with LTGCPU1. 24, 48 or 72 hours later (1), cells were selected on puromycin (10 $\mu$g/mL) for 24 or 48 hours (2). The mean VCN for non-selected (basal) and selected cells was determined for pooled cells and compared with the theoretical expected VCN in optimal selection conditions (Theo).
  **c.d.** Mobilized bone marrow cells were transduced and selected 48 hours later with several concentrations (0-10 $\mu$g/mL) of puromycin during 24 hours. The percentages **(c)** and absolute numbers **(d)** of transduced (grey) and non-transduced (black) erythroid and myeloid progenitors are indicated.

- **Figure 3: LTGCPU7 titer and function**

  **a.** NIH3T3 cells were mock-transduced (NT) or transduced with LTGCPU7, and LTGCPU7-transduced cells were selected with puromycin. Equivalent numbers of mock- and LTGCPU7-transduced/selected cells were treated with GCV at the indicated concentration. Living cells were counted three days later.
  **b.** Five different cord blood CD34$^+$ cell samples were transduced with LTGCPU7, left untreated (-) or treated (+) with 5 $\mu$g/mL puromycin for 24 hours and plated on semi-solid medium. The percentage of erythroid colonies carrying the vector is indicated.

- **Figure 4: Inefficient selection of SRCs is correlated with high level MDR1 in CD34$^+$CD133$^+$ cells.**

  **a.b.c.** Cord blood CD34$^+$ cells were transduced with LTGCPU7, left untreated (-) or treated (+) with puromycin and plated on semi-solid medium or injected into NSG mice. The mean VCN in pooled erythroid cells **(a)** and the proportion of transduced and untransduced progenitor cells **(b)** were determined for erythroid colonies retrieved from methylcellulose. Mice received 30,000 cells (5 mice in the untreated group and 6 mice in the treated group) or 150,000 cells (6 mice in each group). The mean VCN values and their median were determined for human CD45$^+$cells sorted from the bone marrow of individual mice three months after transplantation (c).
  **d.** Five different cord blood CD34$^+$ cell samples were transduced with LTGCPU7 and analyzed for the presence of CD243 (MDR1), CD34, and CD133 antigens on the day of transduction (day 1) and two (day 3) and three (day 4) days later. Results are the mean +/-SD.

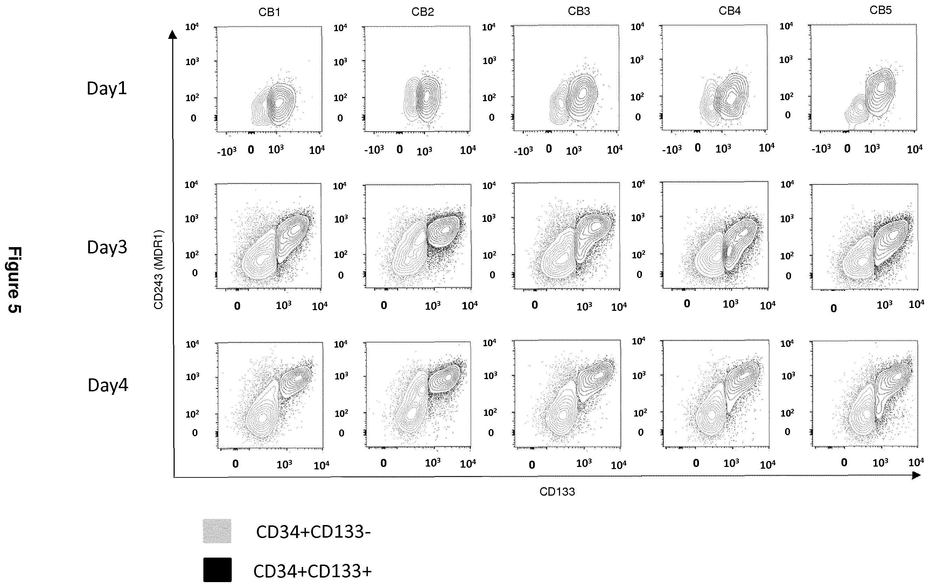

- **Figure 5: Cell culture and MDR1 expression in human hematopoietic CD34$^+$ cells.** Five different cord blood CD34$^+$ cell samples (CB1 to CB5) were transduced with LTGCPU7 and analyzed for the presence of CD243 (MDR1), CD34, and CD133 antigens on the day of transduction (day 1) and two (day 3) and three days (day 4) later. Correlation of MDR1 and CD133 expression in CD34$^+$ cells on days 1, 3, and 4 is shown.

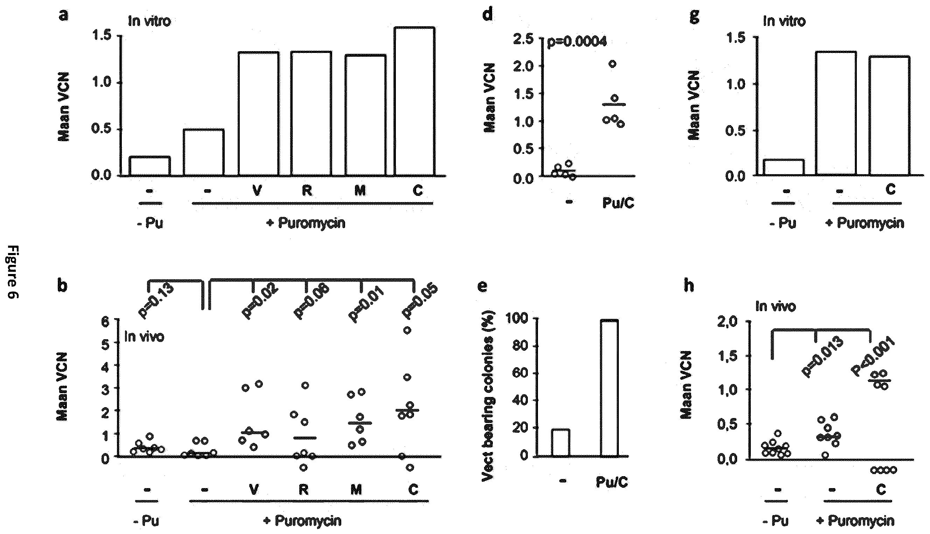

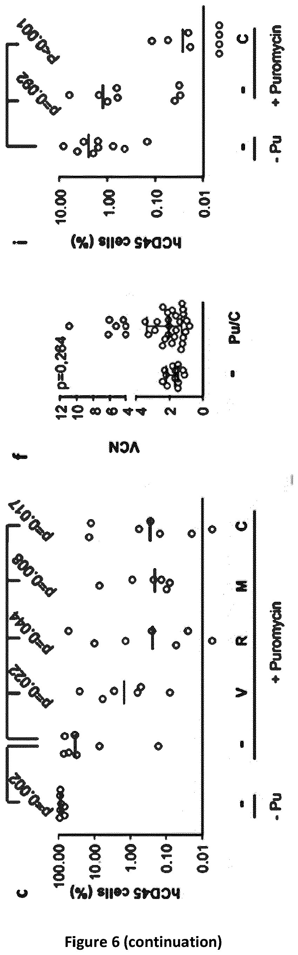

- **Figure 6: Selection of transduced SRCs in the presence of MDR1 inhibitors**

  **a.b.c.** Human cord blood CD34$^+$ cells were transduced with LTGCPU7 and left untreated (-Pu) or treated with 5 $\mu$g/mL puromycin, in the absence (-) or presence of MDR inhibitors (verapamil (V, 20 $\mu$M), reserpine (R, 20 $\mu$M), mifepristone (M, 5 $\mu$M), or cyclosporin A (C, 2 $\mu$M)). Cells were plated on semi-solid medium or injected into NSG mice (65,000 cells per mouse in the treated groups and 225,000 cells *per* mouse in the non-selected group). Mean VCN was determined for pooled cells from *in vitro* culture **(a)** and from human CD45$^+$ cells sorted from the bone marrow of individual mice three months after transplantation **(b)**. The human chimerism in the bone marrow of individual mice was also assessed, by flow cytometry **(c)**. The bars indicate the median value for mean VCN and the level of human chimerism. Levels of chimerism < 0.01% were below the limit of quantification. Mice with such low levels of chimerism (shown below the x-axis) were not included in the calculation of median VCN in **b**.
  **d.e.f.** The experiment was repeated with puromycin and cyclosporin A (2 $\mu$M). Mean VCN was determined in human CD45$^+$cells **(d)** and in human erythroid progenitors retrieved from individual mice **(e,f)**. The proportion of vector-bearing progenitors **(e)** and the VCN in individual colonies **(f)** from NSG mice are indicated. The black bars indicate the median and interquartile values.

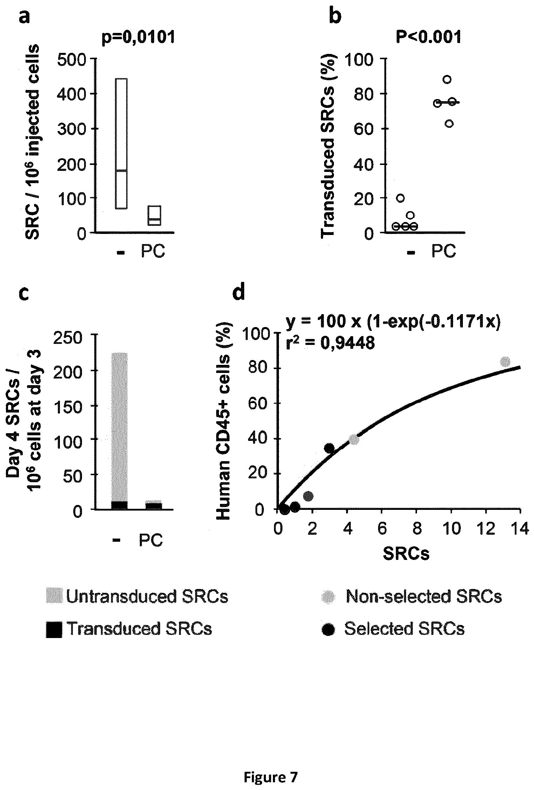

- **Figure 7: Recovery of transduced and selected SRCs**
  Human cord blood CD34$^+$ cells were transduced with LTGCPU7 and either left untreated (-) or treated with puromycin + cyclosporin A (PC). On day 4, six groups of mice received 10,000, 25,000 and 75,000 treated or untreated cells. Three months later, the proportion of hCD45$^+$ cells in the bone marrow was evaluated by flow cytometry, to determine the frequency of SRCs **(a)** and the proportion of transduced SRCs, based on the proportion of their erythroid progeny carrying the vector **(b)**. These data were used to calculate the relative numbers of transduced and non-transduced SRCs **(c)** and the reconstitution capacity of SRCs in NSG mice **(d)**.

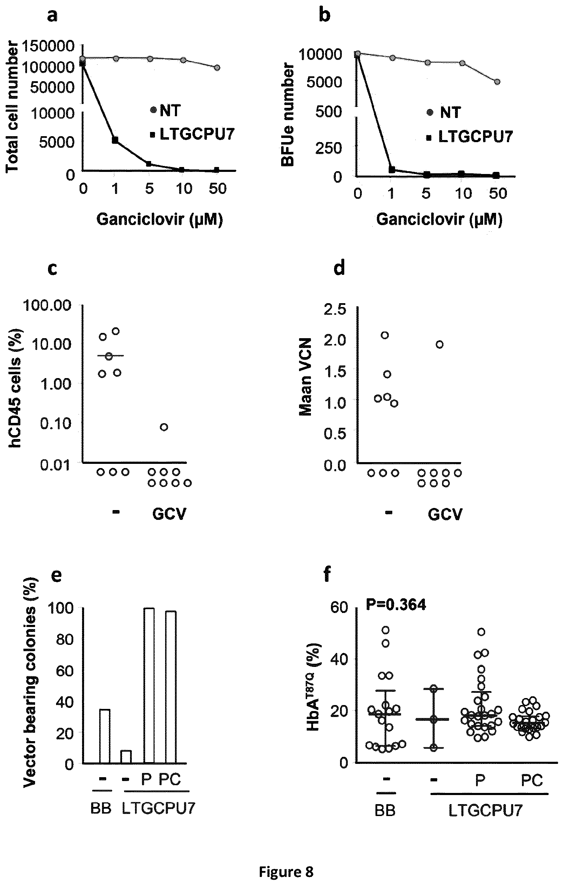

- **Figure 8: Conditional suicide and expression of the $\beta$-globin therapeutic gene**

  **a.b.c.d.** Human cord blood CD34$^+$ cells were transduced with LTGCPU7 and treated with puromycin and cyclosporine A for 24 hours. Non-transduced (NT) and LTGCPU7-transduced cells were then treated with ganciclovir (GCV) for three days, counted **(a)** and plated on semi-solid medium for the evaluation of erythroid progenitor cell eradication **(b)**. Selected cells were also injected into 16 NSG mice, half of which were treated with GCV four weeks later (50 mg/kg, 6 days per week, for 2 weeks). Human chimerism **(c)** and VCN in human CD45$^+$ cells **(d)** were determined 2 months later.
  **e.f.** Human CD34$^+$ cells from a patient with sickle cell anemia were transduced with BB305 or LTGCPU7. LTGCPU7-transduced cells were left untreated (-), were selected with puromycin (P), or selected with puromcyin + cyclosporin A (PC). Transduced cells were plated on semi-solid medium.
  The presence of the integrated vector was assessed in individual colonies **(e)** and the amount of transgenic hemoglobin (HbAT87Q/HbAT87Q+HbS) was then compared **(f)**. The black bars indicate the median and interquartile values.

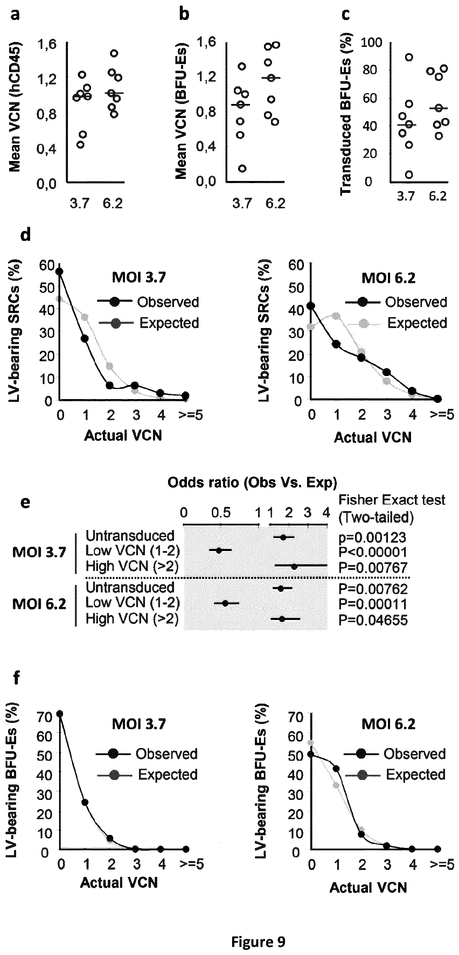

- Figure 9: Lentiviral transduction and comparison with a random distribution
  Human cord blood CD34$^+$ cells were transduced at two different MOI (3.7 and 6.2) with a purified lentiviral LTGCPU7 vector preparation. Cells were injected into NSG mice (75,000 cells per mouse, 7 mice per group). Three months after cell infusion, human CD45+ bone marrow cells were harvested and mean VCN was determined **(a)**. Bone marrow cells were plated on semi-solid medium and VCN was determined in ≈50 individual colonies per animal (≈350 per MOI) to calculate the mean VCN **(b)** and the proportion of vector-bearing cells **(c)** in the progeny of SRCs. The VCN distribution was compared with a Poisson distribution **(d)** and the odds ratio for the differences between the observed and expected numbers of cells bearing particularly numbers of vector copies were calculated **(e)**. Before injection in NSG mice, cells were also plated on semi-solid medium and the VCN was assessed in 50 individual colonies per MOI and compared with the expected values based on a Poisson distribution **(f)**. The bars shown in **a, b** and **c** represent the median values. Dots and bars in **e** indicate the odds ratio and 95% confidence intervals, respectively.

In the following description numerous specific details are set forth in order to provide a thorough understanding. It will be apparent however, to one skilled in the art, that the present invention may be practiced without limitation to these specific details. In other instances, well known methods and structures have not been described, so as not to unnecessarily obscure the description.

## EXAMPLE 1: Materials and Methods

### - Lentiviral vector preparation, purification, titration, and CD34+ cell transduction

The HPV524 vector is similar to the previously described beta-globin lentiviral vector HPV569 (Cavazzana-Calvo, M. et al., Nature, 2010, 467, 318-322) except that it contains no chromatin insulators (cHS4). The PAC and humanphosphoglycerate kinase promoter sequences were synthesized by Genscript (Piscataway, USA) and introduced between the beta-globin locus control region and the 3' polypurine tract of HPV524 to make LTGCPU1. LTGCPU7 is derived from the more recent beta-globin lentiviral vector BB305 (Negre, O. et al. Current gene therapy, 2015, 15, 64-81) (**Figure 1**). In the products of PAC/deltaTK constructs, the two proteins are linked via a glycine-serine spacer (GS) and deltaTK starts at the second ATG of the full-length HSV-TK sequence. The PAC/deltaTK_opt cassette was optimized and synthesized by DNA2.0 (Newark, USA), for optimal expression in human cells. Its sequence, together with that of the EFS promoter, corresponds to SEQ ID NO: 1. Stomatitis virus glycoprotein-pseudotyped lentiviral particles were produced by a cotransfection method (Westerman et al., Retrovirology, 2007, 4, 96) involving four to five plasmids and PEIpro (Polyplus, Illkirch France), concentrated 1000-fold by two rounds of ultracentrifugation at $100,000 \times g$ for 90 minutes in a SW32TI rotor (Beckman Coulter). Where indicated, purification was performed with a Mustang Q anion exchange membrane cartridge (Pall Corporation), as previously described (Kutner et al., Nature protocols, 2009, 4, 495-505) and a 40K ZebaSpin desalting column (ThermoFisher Scientific), according to the manufacturer's instructions, with concentration by ultracentrifugation. Infectious titers were determined by qPCR, as previously described (Payen, E. et al., Methods in enzymology, 2012, 507, 109-124). When indicated, transduced NIH3T3 cells were selected on puromycin and treated with various concentrations of ganciclovir (Cymevan, Roche). Adult and cord blood CD34+ cells were cultured in SCGM (CellGenix) and X-VIVO 20 (Lonza) medium, respectively, and transduced as previously described (Negre, O. et al., Current gene therapy, 2015, 15, 64-81). Cells were selected 48 hours after transduction, on puromycin hydrochloride (5 μg/mL, Sigma-Aldrich). Where indicated, mifepristone, reserpine, cyclosporin A, verapamil hydrochloride, all from Sigma-Aldrich, were used at final concentrations of 5 μM, 20 μM, 2 μM, and 20 μM respectively, together with puromycin.

### - Immunodeficient mice

NOD.Cg-Prkdc[scid] Il2rg[tm1Wjl]/SzJ (NOD-*scid IL2r*gamma[null] abbreviated to NSG) mice were obtained from Charles River Laboratories (Saint Germain sur l'Arbresle, France) and kept in individually ventilated cages supplied with sterile food and autoclaved water. Animal care and handling conformed to EU Directive 2010/63/EU on the protection of animals used for scientific purposes. Experimental protocols were approved by the ethics committee for animal experimentation of the CEA, under notification number 12-033. Three- to four-month-old female recipients (at least 6 mice per group) were sublethally irradiated with a dose of 2.8 Gy (1 Gy/min) to the whole body (TBI). Then, $10^4$ to $10^5$ transduced and selected human cells were injected intravenously into the mice on the day after irradiation. Where indicated, mice were treated, one month after cell infusion, with intraperitoneal ganciclovir (Cymevan, Roche), at a dose of 50 mg/kg/day (or saline) on six days per week, for two weeks. Bone marrow hematopoietic cells were harvested from the left and right femurs and tibias, three months after the transplantation of human cells. They were used for human progenitor assays, flow cytometric analysis and hCD45 cell sorting.

## - Human cells

The study was approved by the ethics evaluation committee of Inserm, institutional review board (IRB00003888) of the French institute of medical research and health (IORG0003254 FWA00005831). Cord blood cells were obtained from Saint-Louis Hospital (Paris, France). Mobilized peripheral blood cells were collected from the residual cells in bags prepared for transplantation at Pitié Salpêtrière Hospital (Paris, France). Adult bone marrow cells were obtained from hip operations at the "Centre Hospitalier Intercommunal" (Créteil, France). The mononuclear cell fractions were isolated by gradient separation and human CD34$^+$ cells were isolated with the CD34$^+$ progenitor cell isolation kit (Miltenyi Biotech) and the autoMACSPro instrument (Miltenyi Biotech), according to the manufacturer's instructions.

## - Progenitor assays and vector copy number determination in human progenitor cells

For progenitor assays performed immediately after *in vitro* transduction and selection, 500 to 1000 cells were plated in methylcellulose based-medium already supplemented with human cytokines (H4434, Stemcell Technologies). For progenitor assays performed with human cells isolated from immunodeficient mice after transplantation, the growth of mouse cells was prevented by plating 100,000 to 1,000,000 cells on methylcellulose-based medium (H4230, Stemcell Technologies) supplemented with human stem cell factor (50 ng/mL, Peprotech), human interleukin 3 (10 ng/mL,Peprotech) and human erythropoietin (3 U/mL). The colonies were counted and collected after incubation for 11-14 days, washed with PBS and stored. DNA was recovered and amplified with the TaqMan Sample-to-SNP kit (ThermoFisher Scientific), specific primers and probes (Table **1**), with the 7300 ABI Prism Detection system (Applied Biosystems).

**Table 1: Primers and probes used for VCN determination in human progenitor cells**

| Assay | Amplicon | Primer and Probe | Sequence (5'-3') | modification | Concentration (nM) |
|---|---|---|---|---|---|
| Vector copy number | Lentiviral vector | HPV569F21 | TGTGTGCCCGTCTGTTGTGT (SEQ ID NO : 2) | - | 900 |
| | | HPV569R19 | CGAGTCCTGCGTCGAGAGA (SEQ ID NO : 3) | - | 900 |
| | | HPV569P2 | CAGTGGCGCCCGAACAGGGA (SEQ ID NO : 4) | 5'FAM 3'BHQ1 | 200 |
| | Human HCK | hHCKF1 | TATTAGCACCATCCATAGGAGGCTT (SEQ ID NO : 5) | - | 900 |
| | | hHCKR1 | GTTAGGGAAAGTGGAGCGGAAG (SEQ ID NO : 6) | - | 900 |
| | | hHCKP3 | TAACGCGTCCACCAAGGATGCGAAT (SEQ ID NO : 7) | 5'YY 3'BHQ1 | 200 |

FAM, 6-carboxyfluorescein ester; TAMRA, tetramethyl-6-carboxyrhodamine; YY, yakima yellow; BHQ1, black hole quencher-1

The results were compared with those obtained with genomic DNA from a human cell line containing a single copy of the integrated lentiviral vector per haploid genome. Vector copy numbers were determined by the comparative Ct method and set at integer values. Colony-forming cells with VCN values between 0.5 and 1.5 were considered to have a VCN of 1; those with VCN values between 1.5 and 2.5 were considered to have a VCN of 2, and so on. LTC-IC cells were analyzed as previously described (Payen, E. et al., Methods in enzymology, 2012, 507, 109-124). The percentage of vector-modified LTC-IC during the readout phase of the assay was determined, by subjecting no more than one clonogenic myeloid colony per well to qPCR-based scoring to ensure that only independent LTC-ICs were analyzed.

- **Flow cytometry**

The antibodies used for cytometry were directed against murine CD45 (eBioscience, clone 30-F11), human CD45 (Miltenyi, clone 5B1), human CD33 (BD Bioscience, clone WM53), human CD15 (Miltenyi, clone VIMC6), human CD20 (BD Bioscience, clone 2H7), human CD3 (Miltenyi, clone BW264/56), human CD133 (Miltenyi, clone AC133), human CD34 (BD Bioscience, clone 581) and human CD243 (eBioscience, clone UIC2). Non-viable cells were excluded by Sytox Blue nucleic acid staining (ThermoFisher Scientific) or with 7AAD (eBioscience). Data were acquired with MACSQUANT instrument (Miltenyi) and analyzed with FlowJo software (Tree Star, Inc). The percentages of human cells in NSG bone marrow were calculated as follows: the number of human CD45 cells was divided by the number of human plus mouse CD45 cells, and the result was multiplied by 100. For limiting dilution assays and the assessment of human stem cell frequency, mice were arbitrarily considered to be positive for human SRCs if the level of human chimerism (human CD45% divided by total CD45%) was >1%, with the presence of both myeloid and lymphoid cells. Mice that had not undergone the transplantation procedure were used as a negative control. Background levels of human cell detection in these negative control mice were ≈0.01%. Isotype control antibodies were used to set the negative gates for the *in vitro* identification of human cell subsets.

- **Human CD45 cell enrichment and vector copy number determination**

Human cells from NSG mouse bone marrow were enriched to facilitate vector copy number (VCN) determination in these cells and to confirm the absence of human cells in immunodeficient mice, as follows: single-cell suspensions of bone marrow cells (20 to 70 million cells) were labeled by incubation with 0.2 $\mu$L whole-blood CD45 microbeads (Miltenyi Biotech) in 50 $\mu$L PBS supplemented with bovine serum albumin (0.5%) and EDTA (2 mM) for 15 minutes, washed, resuspended in 1 mL and sorted with an autoMACSPro instrument (Miltenyi Biotech) and the possel_s program. Sorted cells were then resuspended in PBS for the evaluation of cell enrichment by FACS and DNA extraction with the Nucleospin Blood kit (Macherey Nagel). Relative enrichment varied between 1- and 20-fold, depending on the initial proportion of human cells. In mice with levels of hCD45$^+$ cells below background levels (0.01%), no enrichment was observed, confirming the absence of human cells. Real-time PCR was performed on sorted cells, with a 2X quantitative PCR (qPCR) mastermix containing ROX (Eurogentec) and specific primers and probes (**Table 1**). Vector copy numbers were determined from the results obtained with genomic DNA from a human cell line containing one copy of the integrated lentiviral vector per haploid genome.

- **Transgenic human hemoglobin from individual erythroid colonies**

Cells from erythroid bursts were lysed by adding 100 $\mu$L H$_2$0 and centrifuging at 20,000 $\times$ g. The nuclei were retained for qPCR and the assessment of vector modification, whereas hemoglobin from individual erythroid colony supernatants was separated by ion exchange HPLC on a PolyCAT A column (PolyLC Inc.). Elution was achieved with a linear gradient of buffer A (40 mM Tris, 3 mM KCN; pH adjusted to 6.5 with acetic acid) and buffer B (40 mM Tris, 3 mM KCN, 200 mM NaCl; pH adjusted to 6.5 with acetic acid) of different ionic strengths at a flow rate of 0.4 mL/min at 40°C. The detection wavelength was 418 nm. HPLC analyses were performed with a Prominence chromatograph (Shimadzu) and LC Solution software.

- **Poisson statistics**

- Distribution of vector copy number per cell and percentage of transduced cells in non-selective conditions: If cells from a specific lineage are transducible at a known mean rate ($\mu$), if they are equally transducible, and if integration events occur independently of each other, the number of vector copies per cell (X) is a discrete random variable the scattering of which obeys a Poisson distribution. The probability mass function of X is given by f(i;$\mu$) = Pr(X=i;$\mu$) = (e$^{-\mu}$)($\mu^i$)/i! where i is the discrete number of vectors integrated per cell and $\mu$ is the observed mean VCN. The probability of a cell being transduced Pr(X≥1;μ) = 1-Pr(X=0;μ) = 1-e$^{-μ}$. Thus, if the mean VCN/cell (μ) in a specific cell population is known, and if the distribution of vector copy number per cell follows a Poisson distribution, the expected percentage of transduced cells in non-selective conditions is (1 - e$^{-μ}$)*100.

- Expected mean VCN after the selection of transduced cells in optimal conditions: In conditions of optimal selection, all non-transduced cells are eradicated and all vector-bearing cells survive. If (1) vector distribution before cell selection follows a Poisson distribution and (2) selection occurs in optimal conditions, the expected VCN after selection is:

$$expVCN_{Sel} = ([Pr(X=1;μ)*1]+[Pr(X=2;μ)*2]+......+[Pr(X=n;μ)*n])/(1-Pr(X=0;μ)) =$$

When μ ≥ 5, expVCNsel ≈ μ.

If the distribution of the vector before selection does not obey Poisson statistics, the expected VCN after selection in optimal selective conditions cannot be predicted in this way.

- Expected distribution after the selection of transduced cells in optimal conditions: This distribution is calculated based on the observed distribution before selection. For each discrete number of integrated vectors per cell, Pr(X=i)$_A$ = Pr(X=i)$_B$ / (1-Pr(X=0)$_B$) where the A and B indices relate to values measured after and before selection, respectively.

## - Insertion site retrieval and bioinformatics

Human CD45$^+$ cells were harvested from the bone marrow of mice three months after the infusion of transduced human CD34$^+$ cells. The numbers of insertion site recovery was increased by preparing genomic DNA from these cells and all their progeny (obtained by culture in methylcellulose). The number of *in vivo* insertion sites was calculated as the sum of unique insertion sites retrieved from these two DNA preparations. Before transplantation, aliquots of cells were subjected to culture on methylcellulose, to select committed hematopoietic progenitor cells, from which genomic DNA was prepared. The number of *in vitro* insertion sites was determined with these cells. DNA was extracted with the Nucleospin Blood kit (Macherey Nagel). All primers were purchased from Eurogentec (**Table 1**). Linear amplification-mediated (LAM) PCR was performed essentially as previously described (Schmidt, M. et al., Nature methods, 2007, 4, 1051-1057). Each DNA sample (500 ng) was linearly amplified for 50 cycles with the 5'-biotinylated primer LTR1b and Q5 high-fidelity DNA polymerase (New England Biolabs). Linear PCR products were captured on M-280 streptavidin-coupled Dynabeads (ThermoFisher Scientific), and complementary DNA strands were synthesized with the Klenow fragment of DNA polymerase I (NEB) and hexanucleotides. The resulting double-stranded DNAs were separately digested with *Mlu*CI, *Mse*I or *Hin*P1I, ligated to complementary linker cassettes (LK3, LK4 and LKS, respectively) with the Fast Link DNA ligase (Epicentre), and denatured with sodium hydroxide. *De novo* synthesized (non-biotinylated) single-stranded DNA fragments were purified with a magnetic stand and pooled by sample. Exponential PCR was performed on pooled samples with primers LTRIIIB and LCII, and the Q polymerase, for 35 cycles. PCR products were purifed with NucleoMag NGS beads (Macherey Nagel), in a 1.1:1 ratio, and quantified using the Qubit fluorometric assay (ThermoFisher Scientific). Next-generation sequencing (NGS) libraries were generated by PCR amplification on 40 ng of PCR products with the LCII-SEQ and LTRIV-SEQ primers, for 12 cycles with Q5 polymerase, and were purified with nucleoMag NGS beads used in a 0.8:1 ratio. It was possible to sequence all amplicons in one run, because each library was prepared with a specific LTRIV-SEQ primer, these primers differing by 6 to 12 nucleotides introduced between the Illumina adapter sequence and the nucleotide sequence complementary to the vector LTR. PCR products were analyzed with the 2100 Bioanalyzer high-sensitivity DNA chip (Agilent), quantified with the Qubit assay, mixed in equimolar concentrations with genomic libraries to compensate for low diversity, and sequenced on a NextSeq 500 instrument (Illumina) by the NGS core facility of I2BC (I2BC, CNRS, Gif-sur-Yvette, France). Once the run was completed, the reads were demultiplexed with bcl2fastq2 version 2.15.0 and cutadapt verstion 1.9.1, and FASTQ format files were generated for downstream analysis. Demultiplexed reads were analyzed on a local Galaxy platform, with the Galaxy open-source application (Afgan, E. et al., Nucleic acids research, 2016) and tools obtained from the Galaxy tool shed (Blankenberg, D. et al., Genome biology, 2014, 15, 403). The linker cassette sequence was removed with Trim Galore! (http://www.bioinformatics.babraham.ac.uk/proiects/trim_galore/) and reads were processed with tools from the FASTX-toolkit (http://hannonlab.cshl.edu/fastx_toolkit/). Low -quality base calls (Phred score < 20) were trimmed at the 3' end, and reads of overall poor quality were discarded (Phred score < 25 for at least 10% of their bases). Reads containing the internal vector sequence were identified with the Burrows-Wheeler Aligner (BWA) (Li, H. & Durbin, Bioinformatics, 2009, 25, 1754-1760) and discarded. Actual vector-genome-junction reads were identified by mapping the lentiviral vector LTR with BWA, imposing an overall minimal sequence homology of 89% on the last 42 nucleotides of the LTR and a perfect match on the last three bases. Selected reads were trimmed for the LTR sequence, and reads of >29 bp were aligned with the Hg19/GRCh37 human genome with BWA. The resulting BAM alignment files were filtered with SAMtools to retain only reads mapped with the highest mapping probability (mapping quality score of 37) (Li, H. et al., Bioinformatics, 2009, 25, 2078-2079). The integration site position for each read was retrieved formatted in BED format (Quinlan, A.R. & Hall, Bioinformatics, 2010, 26, 841-842) and integration sites were considered to be identical if their relative alignments began within 3 bp of each other. Collision filtering was performed to assign insertion sites to their correct group of samples, based on a minimal read abundance >90%. A matched random control (MRC) dataset was generated by the random extraction of 3000 start sites of DNA sequences mapped onto the Hg19/GRCh37 human genome, with distances to the closest cutting site for *Miu*CI, *Mse*I, or *Hin*P1I of 20 to 500 bp. The degree of enrichment in integration events around genomic features was compared between the MRC dataset and the insertion sites retrieved from LTGCPU7-transduced cells. Using the BEDTools suite (Quinlan, A.R. & Hall, Bioinformatics, 2010, 26, 841-842) the distance to the closest transcription start sites (TSS) of refseq genes and oncogenes, the intersection with exons and introns, and the densities of transcripts, DNAse I hypersensitive sites (HS) and CpG islands were determined. The genomic coordinates of CpG islands, refseq genes, RNA-seq and DNAse I HS sites were obtained from the University of California Santa Cruz (UCSC) annotation database (http://hgdownload.cse.ucsc.edu/goldenPath/hg19/database) (Speir, M.L. et al., Nucleic acids, 2016, 44, D717-725). The list of oncogenes was retrieved from the allonco data set (http://www.bushmanlab.org/links/genelists) generated by Doctor Rick Bushman (University of Pennsylvania). The DNAse I HS sites of CD34[+] cells were obtained from data generated through the ENCODE project (Consortium, E.P., Nature, 2012, 489, 57-74) (Prof. Stamatoyannopoulos, University of Washington) and archived under UCSC accession number wgEncode EH001885. The expression profile was generated from RNA-seq data generated by the BLUEPRINT Consortium for cord blood hematopoietic stem cells. It has been archived by the European Genome-phenome archive under the reference EGAX00001157677. A full list of the investigators contributing to data generation is available from www.blueprint-epigenome.eu. Funding for the project was provided by the European Union's Seventh Framework Programme (FP7/2007-2013) under grant agreement no 282510 - BLUEPRINT.

**Statistical tests**

For comparisons between two or more groups, Student's t test, Mann-Whitney U tests (if the data were shown to be non-normal in Shapiro-Wilk tests), and one-way ANOVA (possibly based on ranks) were used and degrees of significance were calculated with SigmaPlot 10.0 software. Best-fit curve rising to a maximum were constructed with SigmaPlot. The significance of differences between proportions of cells bearing various numbers of lentiviral vector copies in experimental groups and expectations was assessed by creating 2X2 contingency tables assuming equivalent numbers of cells in the two groups. Non-random associations between variables were assessed by Fisher's exact test. Odds ratio and 95% confidence interval were calculated with GraphPad prism 6.The frequency of LTC-ICs and SRCs and the significance of differences between groups were determined with L-Calc software (StemCell Technologies).

**EXAMPLE 2: Optimal dose and timing for the selection of transduced hematopoietic progenitors**

The LTGCPU1 vector expressing the puromycin N-acetyl-transferase (PAC) gene under the human PGK promoter is derived from a β-globin LV that has been tested in an approved human trial for the gene therapy of the beta-hemoglobinopathies (Cavazzana Calvo et al., Nature, 2010; 467, 318-322; **Figure 1).** LTGCPU1 transduced bone marrow (BM) CD34[+] cells were transferred onto selective medium containing 10 μg/mL puromycin at 24 or 48 hours after transduction. They were incubated for 24 hours, and the frequency of functional hematopoietic progenitor cell subsets was evaluated. The absolute numbers of vector-bearing erythroid progenitors (BFU-Es) were similar in the presence and absence of treatment, regardless of the time allowed for PAC gene expression. However, incubation for two days was required for the myeloid (CFU-GMs) and long-term progenitor cells (LTC-ICs) to acquire full resistance to the antibiotic (**Figure 2a**). Mobilized peripheral blood (mPB) CD34[+] cells were also transduced with LTGCPU1 and treated with puromycin. The mean vector copy number (VCN) per cell was determined for pooled cells and compared with the theoretical VCN obtained in optimal conditions (eradication of all non-transduced cells and survival of all vector-bearing cells). In non-selective conditions, the mean VCN was 0.17. Based on the assumption that vector integration obeys Poisson statistics (see below), optimal selection should give rise to 1.08 copies of the vector per cell. Mean VCN was 1.6 when cells were selected 24 hours after transduction, and close to the expected value (1.08) if puromycin treatment began two or three days after transduction (**Figure 2b**). Selection for one or two days resulted in similar VCN values, suggesting that treatment for 24 hours was sufficient to eliminate most of the non-transduced cells. With this optimized protocol, the proportion of vector-bearing progenitors (myeloid and erythroid) increased from 10-20% to more than 90% following treatment with 5 μg/mL puromycin (**Figure 2c**). The absolute numbers of transduced progenitors were similar in the presence and absence of puromycin (**Figure 2d**), confirming that selection had a minimal toxic impact on vector-bearing progenitors with this procedure.

## EXAMPLE 3: Dual PAC and TK gene expression, vector optimization

To construct a dual PAC and HSV1-TK β-globin lentiviral vector (LTGCPU7), a deleted version of the conditional human herpesvirus 1 (HHV-1) thymidine kinase (deltaTK) suicide gene was fused to the PAC open reading frame (**Figure 1**) and a sequence was designed to optimize expression in hupman cells (PAC/deltaTK_opt). To decrease the size of the vector and increase its titer, the PGK promoter was replaced with the short intron-less version of the human EF1alpha promoter (EFS), and the TAT-dependent U3 promoter/enhancer with the cytomegalovirus (CMV) promoter (Negre, O. et al., Current gene therapy, 2015, 15, 64-81). The titer from 4 different batches of the resulting LTGCPU7 vector was compared with that of the clinical BB305 LV. LTGCPU7 had a functional titer that ranged from 52 to 87% of that of BB305 (**Table 2**).

**Table 2: Side-by-side comparison of BB305 and LTGCPU7 titers from 4 independent production experiment**

| Batch | Mean titers (TU/mL) | | Fold difference |
|---|---|---|---|
| | BB305 | LTGCPU7 | |
| 1 | $2.9 \times 10^5$ | $1.5 \times 10^5$ | 2.0 |
| 2 | $1.5 \times 10^6$ | $1.3 \times 10^6$ | 1.2 |
| 3 | $9.0 \times 10^5$ | $4.9 \times 10^5$ | 1.8 |
| 4* | $5.5 \times 10^7$ | $3.4 \times 10^7$ | 1.6 |
| * After one round of ultracentrifugation | | | |

NIH3T3 cells were transduced with LTGCPU7 vector, and were readily selectable with puromycin. Selected cells were sensitive to ganciclovir (**Figure 3a**). In addition, LTGCPU7-transduced cord blood (CB) progenitors were efficiently selected upon puromycin treatment (**Figure 3b**).

## EXAMPLE 4: Resistance of HSCs to puromycin selection and MDR1 expression

CB CD34$^+$ cells were transduced with LTGCPU7, treated with puromycin, studied *in vitro* and also injected into NSG mice. The mean VCN (**Figure 4a**) and the percentage (**Figure 4b**) of vector-bearing cells were higher in the erythroid progenitors from puromycin treated cells. Conversely, the mean VCN in hCD45$^+$ cells isolated from immunodeficient mice receiving puromycin-treated cells was similar to that in the absence of treatment (**Figure 4c**), indicating an absence of selection at the SCID (severe combined immunodeficiency)-repopulating cell (SRC) level. LTGCPU7-transduced CD34$^+$ cells from five different cord blood donors were analyzed for the presence of multidrug resistance protein 1 (MDR1). The percentage of MDR1$^+$ (CD243$^+$) cells was low one day after thawing (at the time of transduction), but had increased by days 3 and 4 (Figure **4d** and **Table 3**)

**Table 3: Percentage of cells expressing MDR1 in the different cell populations**

| day | CB donor | CD34⁻CD133⁻ | CD34⁺CD133⁻ | CD34⁺CD133⁺ |
|---|---|---|---|---|
| **1** | **CB1** | no such cells | 3.9 | 8.9 |
| | **CB2** | no such cells | 16.9 | 20.8 |
| | **CB3** | no such cells | 7.8 | 23.7 |
| | **CB4** | no such cells | 10.1 | 21.5 |
| | **CB5** | no such cells | 3.5 | 39.1 |
| **3** | **CB1** | 15.1 | 20.5 | 79.8 |
| | **CB2** | 29.3 | 42.0 | 90;9 |
| | **CB3** | 18.6 | 23.1 | 80.3 |
| | **CB4** | 27.1 | 17.1 | 60.6 |
| | **CB5** | 17.6 | 14.5 | 72.2 |

(continued)

| day | CB donor | CD34⁻CD133⁻ | CD34⁺CD133⁻ | CD34⁺CD133⁺ |
|---|---|---|---|---|
| **4** | **CB1** | 22.6 | 24.6 | 96.6 |
| | **CB2** | 33.8 | 45.6 | 97.9 |
| | **CB3** | 25.2 | 25.5 | 93.6 |
| | **CB4** | 31.6 | 21.7 | 90.9 |
| | **CB5** | 27.8 | 22.9 | 86.9 |

The more primitive cells correspond to the CD34⁺CD133⁺ subset (Takahashi, M. et al., Leukemia, 2014, 28, 1308-1315). The percentage of MDR1⁺ cells was higher for the more primitive CD34⁺CD133⁺ subset than for CD34⁺CD133⁻ cells (60-90% versus 15-40% on day 3), and MDR1 and CD133 fluorescence intensities were correlated (**Figure 5**). It was concluded that day 3 to day 4 CD34⁺ cells were heterogeneous for MDR1 expression and that cells with the highest SRC activity, at least for those originating from cord blood, had high levels of MDR1 expression.

### EXAMPLE 5: MDR1 inhibitors release the selection inhibition of immature cells

LTGCPU7 transduced CB CD34⁺ cells were treated with puromycin, in the presence or absence of MDR1 inhibitors. Mean VCN was determined in pooled cells from methylcellulose and in human cells recovered from mouse bone marrow. Selection levels were lower than expected in erythroid progenitors (Mean VCN ≈ 0.5) and higher in the presence of MDR1 inhibitors (Figure **6a**). It was hypothesized that CB progenitors have variable levels of MDR1 expression and of susceptibility to puromycin selection, depending on the sample considered. All NSG mice received 65,000 treated cells or 225,000 untreated cells, corresponding to identical numbers of input cells before selection. In the absence of MDR1 inhibitor, no selection of transduced SRCs was observed (**Figure 6b**). By contrast, in the presence of inhibitors, puromycin was able to select vector-bearing cells. As expected, the decrease in SRC numbers led to lower proportions of human cells in mice receiving selected cells than in mice receiving cells treated with puromycin only (**Fig. 6c**). It was checked that the higher VCN in human cells resulted from a higher proportion of modified SRCs, rather than the selection of a small number of highly modified cells, by determining the proportion of vector-bearing SRCs. LTGCPU7 transduced CB CD34⁺ cells were selected, and injected into NSG mice. As expected, the mean VCN in the hCD45⁺ cells of individual mice increased upon cell selection with puromycin and cyclosporin A (**Figure 6d**). Erythroid progenitors retrieved from seven mice, including three from the non-selected group and four from the selected group, were recovered in the form of erythroid colonies and harvested. The vector was detected in 19% of cells from mice in the non-selected group and 100% of cells from mice in the selected group (12 of 63 colonies and 33 of 33 colonies, respectively; $p=10^{-16}$ in Fisher's exact test) (**Figure 6e**), indicating that vector-bearing SRCs, from which bone marrow erythroid progenitors are derived, had been selected. VCN did not differ significantly between individual BFU-Es (p=0.264), suggesting that the selection of transduced cells did not particularly favor the cells with the highest vector copy numbers (**Figure 6f**). The effect of the strategy on the selection of mPB cells was also investigated. The percentage of transduced progenitor cells in vitro increased from 18% to 100% upon selection with puromycin (data not shown). Mean VCN was, therefore, slightly greater than 1, in puromycin-treated cells (**Figure 6g**). The addition of the MDR1 inhibitor (cyclosporine A) increased mean VCN by a factor of more than seven (p<0.001), consistent with the efficient selection of vector-bearing SRCs (**Figure 6h)**. As expected, the level of reconstitution was lower in mice injected with puromycin-treated SRCs (**Figure 6i**).

### EXAMPLE 6: Recovery rate of transduced stem cells

It was investigated whether the lower percentage of human CD45⁺ cells resulted purely from the elimination of untransduced SRCs, or whether it was also the result of particular toxicity to transduced SRCs, by evaluating the absolute numbers of these cells after selection (day 4), in a limiting dilution assay. Cord blood CD34⁺ cells were transduced, selected, and injected into NSG mice. The frequency of SRCs was 4.4 times higher for control than for treated cells (**Figure 7a**), and cell selection decreased the absolute cell count by a factor of 5.4. Thus, in this experiment, the absolute number of SRCs was 24 times higher for control cells than for selected cells. The median transduction efficacies for the erythroid progeny of these cells were 74.9% for selected cells and 4.2% for control cells (**Figure 7b**). Therefore, the absolute number of vector-bearing SRCs on day 4 was only 1.34 times higher for untreated than for treated cells (**Figure 7c**). Reconstitution levels (median proportion of human cells) as a function of the numbers of SRCs injected showed a similar relationship regardless of selection, consistent with a lack of dependence of culture conditions on cell fitness (**Figure 7d**). The lower percentage of human cells in mice receiving selected cells therefore resulted primarily from the elimination of non-transduced SRCs, with only a marginal effect of the loss of transduced SRCs.

## EXAMPLE 7: Conditional suicide

CB CD34+ cells were transduced with LTGCPU7, they were treated with ganciclovir for 72 hours, and plated on methylcellulose. The toxicity of ganciclovir was limited in non-transduced cells (**Figure 8a and Figure 8b**) and the number of transduced erythroid progenitors decreased by a factor of 200 and 1000 in the presence of 1 $\mu$M and 5 $\mu$M ganciclovir respectively (**Figure 8b**). The transduced cells were injected into 16 NSG mice. Four weeks later, eight of the mice received ganciclovir. Two months later, hCD45+ cells were detected in five of the eight mice in the control group and in one of the eight mice of the treated group, although the number of human cells was very small in this mouse (**Figure 8c**). The absence of hCD45+ cells in mice with levels below the limit of sensitivity (<0.01%) was confirmed by the lack of enrichment observed on hCD45 cell sorting. The vector was detected at a similar copy number in ganciclovir-resistant cells than in cells from mice that did not receive ganciclovir (**Figure 8d**), indicating that ganciclovir treatment eliminated most, but not all, transduced cells.

## EXAMPLE 8: Expression of the beta-globin therapeutic gene

CD34+ cells from an individual with sickle cell disease were transduced with BB305 and LTGCPU7. Transduction levels occurred in 35% and 10% of the erythroid progenitor cells respectively **(Figure 8e)** so that vector copy numbers are expected to be no more than one in about 80% and 95% of transduced progenitors for BB305 and LTGCPU7 vectors respectively (see below). Vector-bearing erythroid colonies were identified and beta$^{AT87Q}$-globin protein levels were compared. LTGCPU7-transduced progenitor cells were efficiently selected (**Figure 8e**) and similar amounts of therapeutic beta-globin protein were produced with the two vectors (**Figure 8f**), suggesting an absence of transcriptional interference or competition between the two promoters.

## EXAMPLE 9: Transduction of hematopoietic cells with purified vector

In the above experiments, vectors were produced and immediately concentrated by two rounds of ultracentrifugation. LTGCPU7 vector titers were between $5\times10^7$ and $2\times10^8$ transducing units per milliliter (TU/mL). All transduction protocols were carried out with 2 million CD34$^+$ cells per mL and a 10% vector preparation (volume/volume) giving a multiplicity of infection (MOI) of 2.5 to 10. It was shown that vector purification could substantially enhance transduction of CD34$^+$ human progenitor cells. CB CD34$^+$ cells were transduced with a purified LTGCPU7 vector batch at MOIs of 3.7 and 6.2, and transduced cells were infused into NSG mice. The mean VCNs measured in hCD45$^+$ cells from individual mice (**Figure 9a**) or in the erythroid progeny of SRCs (**Figure 9b**) were higher than those obtained in experiments performed with crude extracts at similar MOIs and in the absence of selection (**Figure 4c, Figure 6h**). Transduction efficiency in the SRC progeny reached 43.4% and 58.5% (**Figure 9c**) and was higher than observed with the non-purified vector (**Figure 7b**).

## EXAMPLE 10: Distribution of vector copy number

**[0082]** In the absence of selection, the distribution of vector integration into individual cells was compared with the expected values calculated from the mean VCN in the populations concerned, based on a Poisson distribution of single events. In erythroid progenitors derived from transduced CD34$^+$cells, the distribution of vector integration events did not differ significantly from expectations (**Figure 9f**). Conversely, in the progeny of SRCs, the observed distribution of vector integration events differed significantly from that predicted on the basis of Poisson statistics (**Figure 9d**) and from a random distribution (**Figure 9e**). The distribution of vector integration into individual cells after selection was also compared with values calculated in conditions in which all vector-bearing cells survive and all untransduced cells die. The proportion of cells with a VCN of 1 was slightly lower than expected on the basis of the vector distributions obtained before selection, in both erythroid progenitors derived from selected CD34$^+$cells and in the progeny of selected SRCs. A small number of cells with insufficiently strong PAC may therefore have been eliminated during selection on puromycin. Accordingly, the mean VCN in the erythroid progeny was higher ($p<0.001$) in selected cells ($4.8\pm2.9$) than in non-selected SRCs ($3.1\pm1.4$).

## EXAMPLE 11: Localization of sites of integration

Cells in which the vector integrates into genomic features associated with high levels of gene expression may be favored during *in vitro* selection, with further enrichment occurring *in vivo* through insertional oncogenesis. This possibility was investigated by determining whether vector integration sites post-selection and post-transplantation presented any evidence of such enrichment close to genes, particularly those associated with cell growth, or chromatin features associated with high levels of gene expression. In this case human cells harvested from the bone marrow of NSG mice receiving selected and non-selected transduced cells were analyzed. The mean VCN in hCD45[+]cells from mouse bone marrow was 4.6±3.0 in selected cells and 2.5±1.4 in non-selected cells. Before transplantation, aliquots of cells were cultured on methylcellulose. The mean VCN was 1.68±0.01 for selected cells and 0.87±0.01 for non-selected cells. As expected for lentiviral vectors, the proportions and distributions of insertion sites associated with genes, distances to the closest (onco)gene, distances to the closest TSS, gene density, transcript abundance, DNAseI HS density, and CpG island density were significantly different from those obtained for a matched random control data set. No significant difference between non-selected and selected *in vivo* samples was observed for any of the feature investigated including the distance of insertion sites to oncogenes. The potential distribution distortions specifically due to *in vitro* drug selection or *in vivo* expansion was evaluated, through the following comparisons: i) non-selected versus selected samples *in vitro,* to evaluate the effect of drug selection; ii) non-selected *in vitro* versus non-selected *in vivo* samples, to evaluate the role of cell expansion; iii) *in vitro* selected versus *in vivo* selected samples, to determine whether any insertion biases observed were additive. Both drug selection and *in vivo* expansion slightly but significantly distorted insertion site distributions towards regions with a high TSS density, regions with a higher proportion of CpG islands and regions with a high density of DNAse hypersensitive sites. Nevertheless, drug-mediated selection had not biased the distribution of insertion sites beyond the possible bias due to in vivo expansion and the distribution biases were not cumulative.

## DISCUSSION

The properties of a relevant beta-globin lentiviral vector capable of transducing a high proportion of hematopoietic cells with a limited number of insertion hits are described herein. The EFS promoter was used to control PAC/deltaTK expression because it is short, potent enough to express clinically relevant genes (Carbonaro, D.A. et al., Mol. Ther., 2014, 22, 607-622), and is associated with a low transformation potential (Zychlinski, D. et al., Mol. Ther., 2008, 16, 718-725). The LTGCPU7 lentiviral vector was produced almost as efficiently as the parental BB305 vector (<2-fold lower), and the vector expressed both operational PAC and functional TK genes in hematopoietic human progenitor cells. In the culture conditions of this study, which are identical to those used in the clinical setting (Payen, E. et al., Methods in enzymology, 2012, 507, 109-124), MDR1 levels were correlated with the characteristic phenotype of stem cells and increased over time, peaking at the time of selection. In the presence of MDR1 inhibitors (Varma M.V. et al., Pharmacological research, 2003, 48, 347-359), it is shown here that the MDR1 substrate puromycin (Theile D. et al., Analytical biochemistry, 2010, 399, 246-250) is effective to select vector-bearing CB and adult SRCs. Cyclosporin A has been shown to relieve lentiviral restriction blocks independently, but at a higher concentration than used here (Petrillo, C. et al., Mol. Ther., 2015, 23, 352-362). Furthermore, none of the inhibitors tested here was capable of altering the transduction rate at the concentrations indicated, in the absence of puromycin.

With selection, a minimal decrease in the number of transduced cells was observed following treatment, whereas most of the non-transduced cells were effectively removed. The slightly smaller number of transduced SRCs (≈25%) may be due to MDR1 inhibition or the residual toxicity of cyclosporin A. However, although this calcineurin inhibitor has been shown to be cytotoxic to some primary cells, these concentrations were much higher than that used here (Jennings, P. et al., American journal of physiology. Renal physiology, 2007, 293, F831-838; Wolf, A. et al. The Journal of pharmacology and experimental therapeutics, 1997, 280, 1328-1334). Furthermore, the hematopoietic reconstitution potential of Mdr1[-/-] HSCs in mice is not affected (Uchida, N. et al., Experimental hematology, 2002, 30, 862-869). The proportion of human cells after transplantation in NSG mice was correlated with the number of cells injected into the mouse and independent of treatment conditions, indicating that there was no effect on SRC fitness. Alternatively, the integration of the vector into regions of heterochromatin may have driven the production of a limited amount of the resistance protein in a minority of transduced cells, leading to the observed cell loss. The observation in the present study that the proportion of cells with a low VCN is slightly lower than expected after cell selection and the preferential distribution of insertion sites upstream from the TSS in gene-dense regions is consistent with this notion. If this should prove to be the case, then this procedure would also select the cells with the highest probability of expressing their transgenes. The pre-transplantation selection of transduced mouse cells by a fluorescence-based method has been shown to prevent subsequent gene silencing (Kalberer, C.P. et al., Proceedings of the National Academy of Sciences of the United States of America, 2000, 97, 5411-5415). This effect would have the advantage of destroying cells harboring vectors but not expressing the therapeutic gene, thereby increasing treatment efficacy. Importantly, drug-mediated selection had not biased the distribution of insertion sites beyond the possible bias due to *in vivo* expansion and the distribution biases were not cumulative.

The ultracentrifugation of non-purified LVs increases the concentration of toxic compounds and decreases the efficiency of target-cell transduction (Yamada, K. et al., BioTechniques, 2003, 34, 1074-1078, 1080). Therefore, the vector was purified by ion exchange chromatography. This made it possible to transduce hematopoietic cells very efficiently, without the plateau in transduction rate rapidly reached when fetal and adult hematopoietic cells are transduced with crude extracts (Griffin, D.O. & Goff, S.P, Journal of virology, 2015, 89, 8096-8100; Griffin, D.O. & Goff, S.P. Retrovirology, 2016, 13, 14). In this setting, the LTGCPU7 vector preparation yielded a transduction efficacy close to the approximately 50% required here to preclude a high frequency of cells having a high VCN and to prevent the waste of cells. Lentiviral transduction rate and the distribution of the vector in late progenitors were consistent with the expected values calculated from the mean copy number in the bulk population of CD34+cells based on Poisson statistics, as suggested in a previous study (Charrier, S. et al., Gene therapy, 2011, 18, 479-487). The significant departure from an ideal Poisson distribution observed in the progeny of SRCs, however, suggests that transduction efficiency is not equal in cells at the most immature stages, resulting in a large number of vector copies in the subpopulation, most susceptible to transduction, and a smaller number in the most resistant subpopulations. These observations indicate that the initial transduction rate for individual cells or colonies should be monitored carefully, to prevent the generation of subsets of cells with a high VCN. It is shown here that adjusting the initial transduction rate to $\approx$50% and the mean VCN to $\approx$1 limits the frequency of SRC progenies with $\geq$5 lentiviral vector copies to about 1%. Conversely, the effort to transduce a higher proportion of HSCs without selection by increased LV delivery with elevation in the average vector copy number also enhances the risk of oncogenesis from multiple integrants per cell. The decrease in total cell number due to selection may make it necessary to increase the number of cells destined for transplant, either through improved CD34+ cell harvesting or effective ex vivo HSC expansion strategies.

A suicide gene was included to decrease the risks related to the potential genotoxicity of integrative vectors and to make it possible to ablate cells with modified genes if a serious adverse event occurred. The use of the HHV-1 TK suicide gene was investigated, as this strategy has been used in many clinical studies (Greco, R. et al., Frontiers in pharmacology, 2015, 6, 95). DeltaTK was incorporated into the vector, as its smaller size was advantageous. Transduced cells were sensitive to ganciclovir treatment with a 1 $\mu$M solution decreasing the number of hematopoietic progenitors by two orders of magnitude. This level of sensitivity is similar to that seen in transduced cell lines or primary T cells expressing deltaTK (Salomon, B. et al., Molecular and cellular biology, 1995, 15, 5322-5328) or fusion proteins including TK or its hypersensitive mutant form, TKSR39 (Chen, X. et al., Cancer immunology, immunotherapy : CII, 2009, 58, 977-987; Junker, K. et al., Gene therapy, 2003, 10, 1189-1197). Transduced SRCs were almost completely eradicated, a result that compares favorably with those of other *in vivo* studies (Gschweng, E.H. et al., Cancer research, 2014, 74, 5173-5183). Nevertheless a few transduced human cells could be detected in one mouse. The presence of these cells may reflect insufficient drug sensitivity, epigenetic silencing of the transgene, or mutations of the TK gene. Epigenetic silencing may not be an issue as, if it occurs in the vicinity of an oncogene, it would also abolish the risk of an adverse event due to transcriptional activation or splicing modulation. Nevertheless, most of the transduced cells were destroyed. Thus, although non obligatory, the presence of delta TK provides a higher level of safety than the use of a vector without a suicide gene. The protocol ensures efficient selection and high recovery rate of transduced hematopoietic stem/progenitor cells after only 24 hours of exposure to selective agents already approved by medical agencies. The drug-selection method is affordable, rapid, easily scalable, and does not bias the distribution of lentiviral insertion sites beyond the integration preferences observed after transduction of HSCs and reconstitution of hematopoiesis. High transduction rates can be obtained with a low/medium vector copy number per cell and the vector provides a means of eradicating cells containing the modified gene *in vivo* if a serious adverse event occurs. The vector titer is close to that of a vector currently used in clinical trials and the beta-globin expression level is similar. These results taken together indicate that this procedure is suitable for human clinical applications while affording the additional safety of conditional suicide. This strategy is very promising for hematopoietic gene and cell therapy, in particular in subjects with beta$^0$/beta$^0$-thalassemia and severe Sickle Cell Disease.

**Claims**

1. A method of selecting genetically modified hematopoietic stem cells, comprising the steps of:

   a) co-delivering at least: (i) a polynucleotide of interest and/or a genome-editing enzyme and (ii) a positive selection marker which is puromycin N-acetyl transferase or a polynucleotide encoding said marker in expressible form, into a population of CD34+ hematopoietic cells, and
   b) contacting the population of hematopoietic cells obtained in a) with an agent for selecting the marker in a) and with a multidrug resistance 1 (MDR1) inhibitor, wherein the agent is puromycin, thereby selecting genetically modified hematopoietic stem cells.

2. The method of claim 1, wherein said CD34+ hematopoietic cells are human.

3. The method of claim 1 or claim 2, wherein the polynucleotide of interest comprises a sequence encoding a protein of interest in expressible form or a sequence which repairs a mutation in a gene of interest.

4. The method of any one of claims 1 to 3, wherein step a) comprises the co-delivery of at least: (i) a polynucleotide of interest or a genome-editing enzyme and (ii) a positive selection marker.

5. The method of any one of claims 1 to 3, wherein step a) comprises the co-delivery or at least a polynucleotide of interest and a polynucleotide encoding the positive selection marker in expressible form, said polynucleotides being inserted in the same vector or in separate vectors.

6. The method of claim 5, wherein at least the polynucleotide of interest is inserted in a lentiviral vector.

7. The method of any one of claims 1 to 6, wherein said MDR1 inhibitor is selected from the group consisting of: cyclosporine A, verapamil, reserpine and mifepristone.

8. The method of any one of claims 3 to 7, wherein the protein of interest is a therapeutic protein.

9. The method of claim 8, wherein said therapeutic protein is human beta-globin.

10. The method of claims 1 to 9, which is carried out in a period of time inferior to one week, preferably four days.

**Patentansprüche**

1. Verfahren zur Selektion von genetisch modifizierten hämatopoetischen Stammzellen, das die folgenden Schritte umfasst:

a) gemeinsame Zufuhr von mindestens: (i) einem Polynukleotid von Interesse und/oder einem Genom-Editing-Enzym und (ii) einem positiven Selektionsmarker, bei dem es sich um Puromycin-N-Acetyl-Transferase handelt oder einem Polynukleotid, das für diesen Marker in exprimierbarer Form kodiert, in eine Population von CD34+ hämatopoetischen Zellen, und
b) Inkontaktbringen der in a) erhaltenen Population hämatopoetischer Zellen mit einem Mittel zur Selektion des Markers in a) und mit einem Multidrug-Resistenz 1 (MDR1)-Inhibitor, wobei das Mittel Puromycin ist, wodurch genetisch modifizierte hämatopoetische Stammzellen selektiert werden.

2. Verfahren nach Anspruch 1, wobei die hämatopoetischen CD34+ Zellen menschlich sind.

3. Verfahren nach Anspruch 1 oder Anspruch 2, wobei das Polynukleotid von Interesse eine Sequenz umfasst, die ein Protein von Interesse in exprimierbarer Form kodiert, oder eine Sequenz, die eine Mutation in einem Gen von Interesse repariert.

4. Verfahren nach einem der Ansprüche 1 bis 3, wobei Schritt a) die gemeinsame Zufuhr von mindestens: (i) einem Polynukleotid von Interesse oder einem Genom-Editing-Enzym und (ii) einem positiven Selektionsmarker umfasst.

5. Verfahren nach einem der Ansprüche 1 bis 3, wobei Schritt a) die gemeinsame Zufuhr mindestens eines Polynukleotids von Interesse und eines Polynukleotids, das für den positiven Selektionsmarker exprimierbarer Form kodiert, wobei die Polynukleotide in denselben Vektor oder in getrennte Vektoren eingefügt werden.

6. Verfahren nach Anspruch 5, wobei zumindest das Polynukleotid von Interesse in einen lentiviralen Vektor eingefügt wird.

7. Verfahren nach einem der Ansprüche 1 bis 6, wobei der MDR1-Inhibitor ausgewählt ist aus der Gruppe bestehend aus: Cyclosporin A, Verapamil, Reserpin und Mifepriston.

8. Verfahren nach einem der Ansprüche 3 bis 7, wobei das Protein von Interesse ein therapeutisches Protein ist.

9. Verfahren nach Anspruch 8, wobei das therapeutische Protein menschliches beta-Globin ist.

10. Verfahren nach einem der Ansprüche 1 bis 9, das in einer Zeitspanne von weniger als einer Woche durchgeführt wird, vorzugsweise vier Tage.

**Revendications**

1. Procédé de sélection de cellules souches hématopoïétiques génétiquement modifiées, comprenant les étapes de :

   a) la co-administration d'au moins : (i) un polynucléotide d'intérêt et/ou une enzyme d'édition génomique et (ii) un marqueur de sélection positive qui est la puromycine N-acétyltransférase ou un polynucléotide codant pour ledit marqueur sous forme exprimable, dans une population de cellules hématopoïétiques CD34+, et
   b) la mise en contact de la population de cellules hématopoïétiques obtenue en a) avec un agent pour la sélection du marqueur en a) et avec un inhibiteur de la résistance multiple aux médicaments 1 (MDR1), dans lequel l'agent est la puromycine, ce qui sélectionne des cellules souches hématopoïétiques génétiquement modifiées.

2. Procédé selon la revendication 1, dans lequel lesdites cellules hématopoïétiques CD34+ sont humaines.

3. Procédé selon la revendication 1 ou la revendication 2, dans lequel le polynucléotide d'intérêt comprend une séquence codant pour une protéine d'intérêt sous forme exprimable ou une séquence qui répare une mutation dans un gène d'intérêt.

4. Procédé selon l'une quelconque des revendications 1 à 3, dans lequel l'étape a) comprend la co-administration d'au moins : (i) un polynucléotide d'intérêt ou une enzyme d'édition génomique et (ii) un marqueur de sélection positive.

5. Procédé selon l'une quelconque des revendications 1 à 3, dans lequel l'étape a) comprend la co-administration d'au moins un polynucléotide d'intérêt et d'un polynucléotide codant pour le marqueur de sélection positive sous forme exprimable, lesdits polynucléotides étant insérés dans le même vecteur ou dans des vecteurs séparés.

6. Procédé selon la revendication 5, dans lequel au moins le polynucléotide d'intérêt est inséré dans un vecteur lentiviral.

7. Procédé selon l'une quelconque des revendications 1 à 6, dans lequel ledit inhibiteur de MDR1 est sélectionné parmi le groupe constitué de : la cyclosporine A, le vérapamil, la réserpine et la mifépristone.

8. Procédé selon l'une quelconque des revendications 3 à 7, dans lequel la protéine d'intérêt est une protéine thérapeutique.

9. Procédé selon la revendication 8, dans lequel ladite protéine thérapeutique est la bêta-globine humaine.

10. Procédé selon les revendications 1 à 9, qui est effectué dans une durée inférieure à une semaine, de préférence quatre jours.

Figure 1

Figure 2

Figure 3

Figure 4

Figure 5

26

Figure 6

Figure 6 (continuation)

**a**

p=0,0101

SRC / 10⁶ injected cells

**b**

P<0.001

Transduced SRCs (%)

**c**

Day 4 SRCs / 10⁶ cells at day 3

**d**

$y = 100 \times (1-exp(-0.1171x))$
$r^2 = 0,9448$

Human CD45+ cells (%)

SRCs

▨ Untransduced SRCs
■ Transduced SRCs

◉ Non-selected SRCs
● Selected SRCs

Figure 7

Figure 8

Figure 9

**REFERENCES CITED IN THE DESCRIPTION**

**Patent documents cited in the description**

- WO 2012094193 A

**Non-patent literature cited in the description**

- **ADAIR et al.** *Sci. Transl. Med.,* 2012, vol. 4, 133ra57
- **PERSONS et al.** *Blood,* 2004, vol. 103, 796
- **DORMIANI et al.** *Gene Therapy,* 2015, vol. 22, 663-674
- **RIBEIL et al.** *New England Journal of Medicine,* 2017, vol. 376, 848-855
- **NEGRE et al.** *Human Gene Therapy,* 2016, vol. 27, 148-165
- **LICHT et al.** *Gene therapy,* 2000, vol. 7, 348-358
- **THEILE et al.** *Analytical Biochemistry,* 2010, vol. 239, 246-250
- **NEGRE, O. et al.** *Human gene therapy,* 2016, vol. 27, 148-165
- **CAVAZZANA-CALVO, M. et al.** *Nature,* 2010, vol. 467, 318-322
- **NEGRE, O. et al.** *Current gene therapy,* 2015, vol. 15, 64-81
- **WESTERMAN et al.** *Retrovirology,* 2007, vol. 4, 96
- **KUTNER et al.** *Nature protocols,* 2009, vol. 4, 495-505
- **PAYEN, E. et al.** *Methods in enzymology,* 2012, vol. 507, 109-124
- **SCHMIDT, M. et al.** *Nature methods,* 2007, vol. 4, 1051-1057
- **AFGAN, E. et al.** *Nucleic acids research,* 2016
- **BLANKENBERG, D. et al.** *Genome biology,* 2014, vol. 15, 403
- **LI, H. ; DURBIN.** *Bioinformatics,* 2009, vol. 25, 1754-1760
- **LI, H. et al.** *Bioinformatics,* 2009, vol. 25, 2078-2079
- **QUINLAN, A.R. ; HALL.** *Bioinformatics,* 2010, vol. 26, 841-842
- **SPEIR, M.L. et al.** *Nucleic acids,* 2016, vol. 44, D717-725
- **CONSORTIUM, E.P ; STAMATOYANNOPOULOS.** Nature. University of Washington, 2012, vol. 489, 57-74
- **CAVAZZANA CALVO et al.** *Nature,* 2010, vol. 467, 318-322
- **TAKAHASHI, M. et al.** *Leukemia,* 2014, vol. 28, 1308-1315
- **CARBONARO, D.A. et al.** *Mol. Ther.,* 2014, vol. 22, 607-622
- **ZYCHLINSKI, D. et al.** *Mol. Ther.,* 2008, vol. 16, 718-725
- **VARMA M.V. et al.** *Pharmacological research,* 2003, vol. 48, 347-359
- **THEILE D. et al.** *Analytical biochemistry,* 2010, vol. 399, 246-250
- **PETRILLO, C. et al.** *Mol. Ther.,* 2015, vol. 23, 352-362
- **JENNINGS, P. et al.** *American journal of physiology. Renal physiology,* 2007, vol. 293, F831-838
- **WOLF, A. et al.** *The Journal of pharmacology and experimental therapeutics,* 1997, vol. 280, 1328-1334
- **UCHIDA, N. et al.** *Experimental hematology,* 2002, vol. 30, 862-869
- **KALBERER, C.P. et al.** *Proceedings of the National Academy of Sciences of the United States of America,* 2000, vol. 97, 5411-5415
- **YAMADA, K. et al.** *BioTechniques,* 2003, vol. 34, 1074-1078, 1080
- **GRIFFIN, D.O ; GOFF, S.P.** *Journal of virology,* 2015, vol. 89, 8096-8100
- **GRIFFIN, D.O. ; GOFF, S.P.** *Retrovirology,* 2016, vol. 13, 14
- **CHARRIER, S. et al.** *Gene therapy,* 2011, vol. 18, 479-487
- **GRECO, R. et al.** *Frontiers in pharmacology,* 2015, vol. 6, 95
- **SALOMON, B. et al.** *Molecular and cellular biology,* 1995, vol. 15, 5322-5328
- **CHEN, X. et al.** *Cancer immunology, immunotherapy : CII,* 2009, vol. 58, 977-987
- **JUNKER, K. et al.** *Gene therapy,* 2003, vol. 10, 1189-1197
- **GSCHWENG, E.H. et al.** *Cancer research,* 2014, vol. 74, 5173-5183